(19) Europäisches Patentamt
European Patent Office
Office européen des brevets

(11) Publication number: **0 214 600 B1**

(12) **EUROPEAN PATENT SPECIFICATION**

(45) Date of publication of patent specification: **02.12.92**

(51) Int. Cl.[5]: **C07D 501/46**, A61K 31/545

(21) Application number: **86112120.0**

(22) Date of filing: **02.09.86**

The file contains technical information submitted after the application was filed and not included in this specification

(54) **Cephalosporin derivatives.**

(30) Priority: **03.09.85 JP 194385/85**
**10.05.86 JP 107262/86**

(43) Date of publication of application:
**18.03.87 Bulletin 87/12**

(45) Publication of the grant of the patent:
**02.12.92 Bulletin 92/49**

(84) Designated Contracting States:
**AT BE CH DE FR GB IT LI NL SE**

(56) References cited:
**EP-A- 0 062 321**
**EP-A- 0 137 441**

**PATENT ABSTRACTS OF JAPAN, vol. 7, no. 69 (C-158)[1214], 23rd March 1983**

(73) Proprietor: **Otsuka Kagaku Kabushiki Kaisha**
**10, Bungo-machi Higashi-ku**
**Osaka-shi Osaka-fu(JP)**

(72) Inventor: **Matsumura, Kiyotoshi**
**463, Kagasuno Kawauchi-cho**
**Tokushima-shi Tokushima-ken(JP)**
Inventor: **Akagi, Hiroshi**
**463, Kagasuno Kawauchi-cho**
**Tokushima-shi Tokushima-ken(JP)**
Inventor: **Suzuki, Daisuke**
**463, Kagasuno Kawauchi-cho**
**Tokushima-shi Tokushima-ken(JP)**
Inventor: **Shimabayashi, Akihiro**
**463, Kagasuno Kawauchi-cho**
**Tokushima-shi Tokushima-ken(JP)**

(74) Representative: **Barz, Peter, Dr. et al**
**Patentanwälte Dr. V. Schmied-Kowarzik**
**Dipl.-Ing. G. Dannenberg Dr. P. Weinhold Dr. D. Gudel Dipl.-Ing. S. Schubert Dr. P. Barz**
**Siegfriedstrasse 8**
**W-8000 München 40(DE)**

**EP 0 214 600 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a novel cephalosporin compound substituted by an imidazolium ring in 3-position of cephem having the formula (I) or (I') and the pharmaceutically acceptable salts thereof:

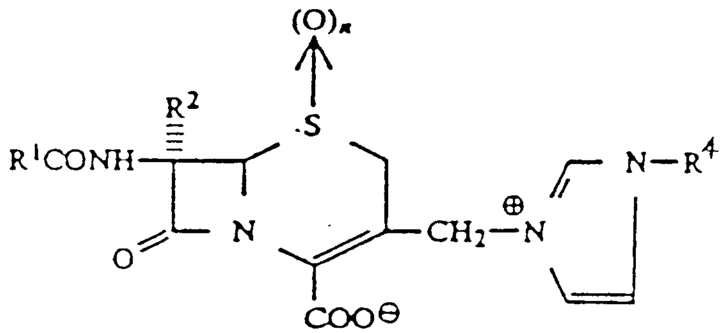

(I)

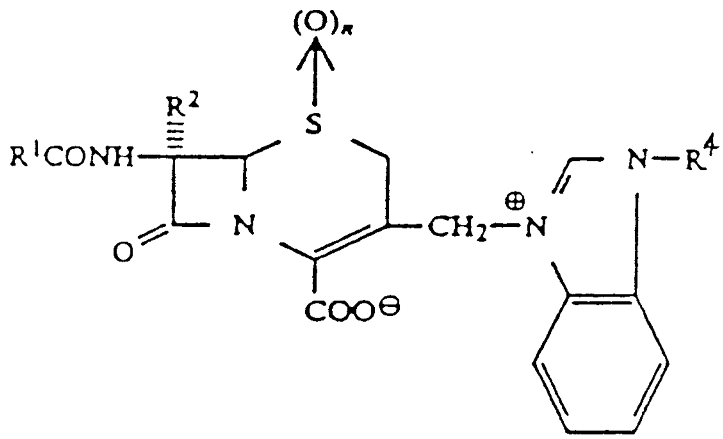

(I')

wherein

$R^1$ is

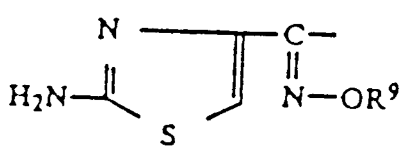

;

$R^2$ is hydrogen or methoxy;

$R^4$ is $C_2$-$C_{12}$ alkenyl, $C_2$-$C_{12}$ alkynyl, benzyl or phenyl;

$R^9$ is $C_1$-$C_6$ alkyl, $C_2$-$C_6$ alkenyl, $C_2$-$C_6$ alkynyl or a group of formula

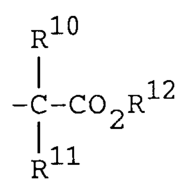

wherein $R^{10}$ and $R^{11}$ are the same or different and represent hydrogen, methyl or ethyl and $R^{12}$ is hydrogen, an alkali metal, alkaline earth metal, organic amine base or protective group for carboxyl; and

n is 0 or 1.

The invention also covers the following compounds 7-[2-(2-Aminothiazole-4-yl)-2-(2-propenyloxyimino)-acetamide]-3-(3-acetophenyl-1-imidazoliomethyl)-3-cephem-4-carboxylate;
7-[2-(2-Aminothiazole-4-yl)-2-(2-propenyloxyimino)-acetamide]-3-(3-(4-hydroxyphenyl)-1-imidazoliomethyl)-3-cephem-4-carboxylate;
7-{2-(2-Aminothiazole-4-yl)-2-[potassium-(2,2-dimethylacetate)oxyimino]acetamide}-3-(3-acetophenyl-1-imidazoliomethyl)-3-cephem-4-carboxylate;
7-{2-(2-Aminothiazole-4-yl)-2-[potassium-(2,2-dimethylacetate)oxyimino]acetamide}-3-(3-(4-hydroxyphenyl)-1-imidazoliomethyl)-3-cephem-4-carboxylate.

The present invention also relates to processes for preparing these compounds and to pharmaceutical compositions containing these compounds.

The compounds of the present invention are useful as pharmaceuticals. A particular aspect of the present invention is their use in the manufacture of a medicament for preventing and treating bacterial infections.

Among the compounds of the present invention, there may be at least one stereoisomer such as an optical isomer due to the presence of asymmetric atom(s) in the molecule. The present invention includes such isomers.

Examples of useful pharmaceutically acceptable salts are alkali metal salts (e.g. sodium salt, potassium salt), alkaline earth metal salts (e.g. calcium salt, magnesium salt), ammonium salt, organic amine salts (e.g. triethylamine salt, pyridinium salt), inorganic acid addition salts (e.g. hydrochloride, hydrobromide), organic acid addition salts (e.g. formate, trichloroacetate, methanesulfonate) and salts with a basic or acidic amino acid (e.g. arginine salt, aspartic acid salt, glutamic acid salt).

In the formula given for radical $R^1$, $R^9$ is selected from methyl, ethyl, propyl, isopropyl, butyl, isobutyl, tert-butyl and like $C_1$~ $C_6$ alkyl; vinyl, propenyl, isopropenyl, butenyl, isobutenyl, pentenyl, hexenyl and like $C_2$~$C_6$ alkenyl; acetylenyl, propargyl and like $C_2$~$C_6$ alkynyl; or a group

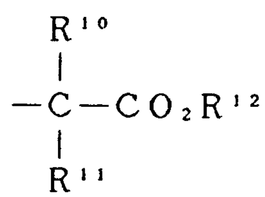

wherein $R^{10}$ and $R^{11}$ are same or different and are hydrogen, methyl or ethyl and $R^{12}$ is hydrogen atom; monovalent alkali metal such as sodium, potassium or lithium, preferably sodium or potassium; divalent alkaline earth metal such as calcium or magnesium; organic amine salts such as trimethylamine, triethylamine, methylamine, propylamine, N,N-dimethylethanolamine; a protective group for carboxyl such as tert-butyl, benzhydryl, 2,2,2-trichloroethyl, p-methoxybenzyl, p-nitrobenzyl, trimethylsilyl, methoxymethyl, benzyloxymethyl, dephenylmethane or phenacyl.

In the following, B is one of the following carbon-containing groups constituting an imidazolium ring:

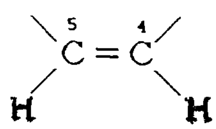

or

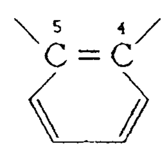

$R^{14}$ is a hydrogen atom; metal atom such as sodium, potassium, etc; ester residue; salt-forming cation; or an anion charge when $COO^-$ forms an intra- or inter-molecular salt with a cation pair. Examples of ester residues are methyl, ethyl, propyl, isopropyl, butyl, isobutyl, tert-butyl and like $C_{1\sim6}$ lower alkyl; 2,2,2-trichloroethyl and like lower haloalkyl; benzyl, p-methoxybenzyl, trimethoxybenzyl, trimethoxydichlorobenzyl, p-nitrobenzyl, benzhydryl and like substituted or unsubstituted phenylalkyl; a protective group for carboxyl such as diphenylmethyl, trityl, ditolylmethyl, phenyl-p-methoxyphenylmethyl, $\alpha$-p-methoxyphenylethyl, $\alpha$-p-methoxyphenyl-$\beta$-trichloroethyl, $\alpha$-diphenylethyl, trimethylsilyl, benzyloxymethyl or phenacyl.

$R^4$ is $C_2$-$C_{12}$ alkenyl such as vinyl, allyl, isopropenyl, butenyl, methallyl; $C_2$~$C_{12}$ alkynyl such as propargyl; benzyl or phenyl.

The cephalosporin compounds and salts thereof of the invention can be prepared by various methods. Preferred methods are shown by the following two synthetic routes.

[ Synthetic route－1 ]

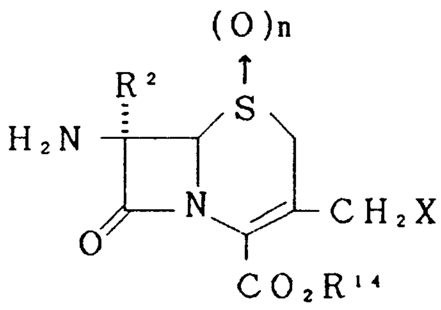

+

$R^1COOH$

(Ⅶ)

or a reactive derivative in amino group, or salt thereof

(Ⅵ)

or a reactive derivative in carboxyl group, or salt therof

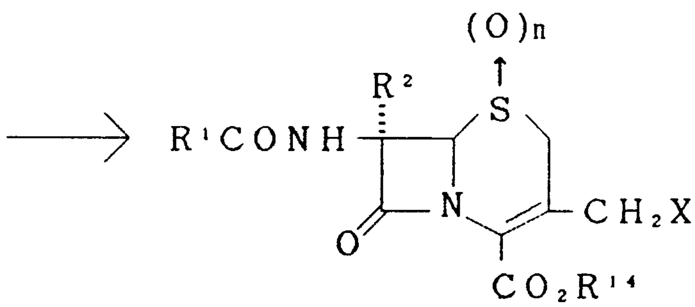

(Ⅱ)

or salt therof

wherein $R^1$ and $R^2$ are same as above, $R^{14}$ is hydrogen atom, metal atom, ester residue, salt-forming cation, or amion charge when $COO^-$ forms intra- or inter-molecular salt with cation pair, n is 0 or 1 , X is a group which can be replaced by a substituted or unsubstituted imidazole compound.

Compound (II) or salt thereof can be obtained by reacting Compound (VII), a reactive derivative in amino group or salt thereof and Compound (VI), a reactive derivative in carboxyl group or salt thereof. As salts of Compounds (VII) and (VI) are used the same salts enumerated with respect to Compound (I). Examples of reactive derivatives of Compound (VII) in amino group are a silyl derivative obtained from Compound (VII) and a silyl compound [eg, bis(trimethylsilyl)acetamide, trimethylsilyl chloride, etc], a derivative obtained from Compound (VII) and an isocyanate or isothiocyanate compound, a Schiff base or its enamine-type tautomer formed by the reaction of Compound (VII) and a carbonyl compound [eg, acetaldehyde, benzaldehyde and like aldehydes, acetone, methyl ethyl ketone and like ketones], etc.

Examples of reactive derivatives of Compound (VI) in carboxyl group are an acid chloride, acid bromide and like acid halides; an acid anhydride or symmetric acid anhydride with a substituted phosphoric acid, dialkylphosphorous acid, sulfurous acid, thiosulfuric acid, sulfuric acid, alkyl carbonate, organic carboxylic acid and like acids; an activated acid amide with an imidazole, dimethyl pyrazole, etc; an activated ester such as p-nitrophenyl ester, phenylthioester, carboxymethyl thioester, an ester with an N-hydroxypiperidine, N-hydroxysuccinimide, N-hydroxyphthalimide and like N-hydroxyl compounds; etc.

In the reaction is usable a solvent such as water, acetone, dioxane, acetonitrile, chloroform, methylene chloride, benzene, ethyl acetate, N,N-dimethylformamide, pyridine, hexane, etc, which does not affect the reaction. The solvent can be used singly or in admixture. The reaction temperature is not particularly limited and the reaction can be conducted under cooling or heating and preferably at about -20 to +40 °C.

The reaction can be conducted in the presence of an organic or inorganic base. Examples of useful bases are lithium, sodium, potassium and like alkali metals; calcium, magnesium and like alkaline earth metals; sodium hydroxide and like alkali metal hydroxides; sodium hydride and like alkali metal hydrides; calcium hydride and like alkaline earth metal hydrides; sodium carbonate and like alkali metal carbonates; potassium hydrogen carbonate and like alkali metal hydrogen carbonates; sodium ethoxide and like alkali metal alkoxides; triethylamine and like trialkylamime; pyridine, picoline, quinoline and like nitrogen-containing heterocyclic compounds; etc. Among these bases, preferably used are trialkylamine and nitrogen-containing heterocyclic compound. The amounts of the base to be used are not particularly limited but are usually up to 25 equivalents, preferably 0.25 to 4 equivalents per equivalent of Compound (VII).

In the above reaction, the proportions of Compound (VII) and Compound (VI) to be used are not particularly limited and are selected from a wide range. However, it is preferable to use in the equivalent ratio of the former : the latter of 1:5 to 5:1, more preferably of 1:2 to 2:1.

In case Compound (VI) is used in the form of free acid or salt thereof, the reaction is conducted preferably in the presence of a condensing agent. Examples of useful condensing agents are N,N'-dicyclohexyl carbodiimide and like carbodiimide compounds; diphenyl ketene-N-cyclohexylimine and like ketene-imine compounds; ethoxyacetylene, β-chlorovinyl ethyl ether and like unsaturated alkyl ether compounds; sulfonic acid ester of N-hydroxybenzotriazole derivative [eg, 1-(4-chlorobenzenesulfonyloxy)-6-chloro-1H-benzotriazole etc]; trialkylphosphite or triphenylphosphine with carbon tetrachloride, ethyl polyphosphate, phosphorus trichloride and like phosphorus compounds; thionyl chloride; Vilsmeyer reagent (formed from the reaction of N,N-dimethyl formamide, N-methyl formamide and like amide compound and thionyl chloride, phosphoryl chloride, phosgene and like halogen compound); etc. The above condensing agent is used usually in an amount of up to 25 equivalents, preferably 0.25 to 4 equivalents per equivalent of Compound (VII).

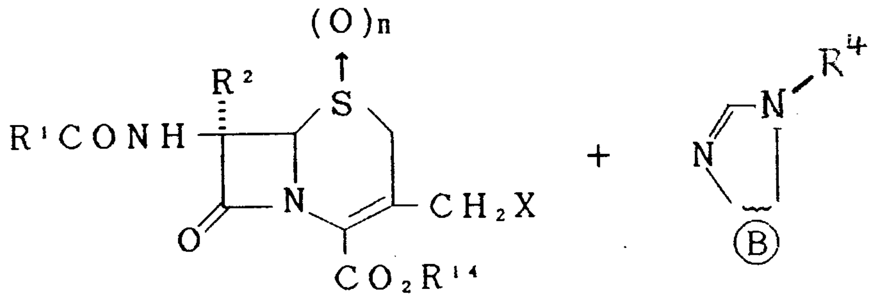

(II) (III)

or salt thereof

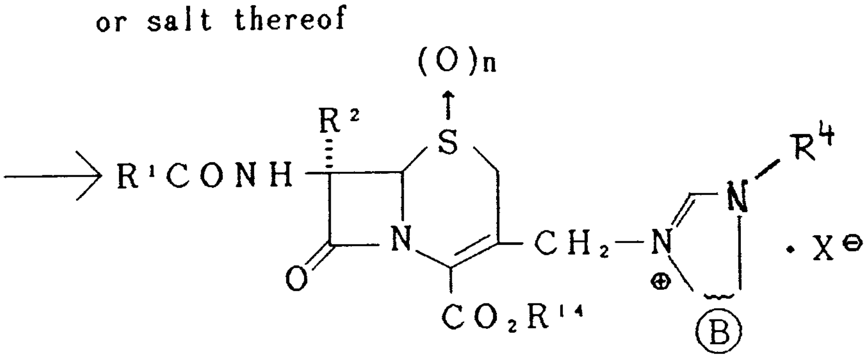

(IV)

or salt thereof

wherein $R^1$, $R^2$, $R^4$, $R^{14}$, B, $X^{\ominus}$ and n are same as above.

The reaction of replacing C-3' position of Compound (II) by substituted or unsubstituted imidazoles (III) is preferably conducted in water or an organic solvent in the presence of a base or propylene oxide.

Examples of organic solvents are acetonitrile, methanol, acetone, N,N-dimethyl formamide, tetrahydrofuran, etc. and these are used singly or in mixture thereof. The base includes sodium carbonate, sodium hydrogen carbonate, triethylamine, etc. The reaction temperature is not particularly limited but the reaction is carried out usually under cooling or heating and preferably at a temperature of about -10 ° C to 40 ° C.

When $R^1$ or $R^{14}$ in Compound (IV) is amino-protecting group or carboxyl-protecting group, or when n is 1, Compound (I) is obtained by the reaction of removing amino-protecting group or carboxyl-protecting group, or by the reduction, respectively. The elimination reaction and reduction can be conducted by a usual manner such as by hydrolysis, reduction, etc. as shown below.

(A) Hydrolysis

The hydrolysis is conducted preferably in the presence of an acid. Examples of useful acids are hydrochloric acid, hydrobromic acid, sulfuric acid and like inorganic acids; formic acid, acetic acid, trifluoroacetic acid, propionic acid, benzenesulfonic acid, p-toluenesulfonic acid and like organic acids.

The hydrolysis is usually conducted in water, methanol, tetrahydrofuran, N,N-dimethyl formamide, dioxane, benzene, hexane and the like, or mixtures of such solvents, and which does not affect the reaction. In case the acid is liquid, the acid can be used as a solvent.

The reaction temperature is not particularly limited but the hydrolysis is preferably conducted under cooling or heating.

(B) Reduction

The reduction includes usual methods such as chemical reduction, catalytic reduction, etc.

Examples of chemical reducing agents are a combination of metal or metallic compound such as tin, zinc, iron, chromium chloride, chromium acetate and organic or inorganic acid such as formic acid, acetic acid, trifluoroacetic acid, p-toluenesulfonic acid, hydrochloric acid, hydrobromic acid; phosphorus tribromide; etc.

Catalysts used in the catalytic reduction include usual one containing a heavy metal such as platinum, palladium, rhodium, nickel, copper, cobalt, iron, etc.

The catalytic reduction is usually conducted in water, methanol, N, N-dimethyl formamide, hexane, benzene, dioxane and the like, or mixtures of such solvents, and which does not affect the reaction. In case the acid is liquid, the acid can be used as a solvent.

The reaction temperature is not particularly limited but the catalytic reduction is preferably conducted under cooling or heating.

[ Synthetic route-2 ]

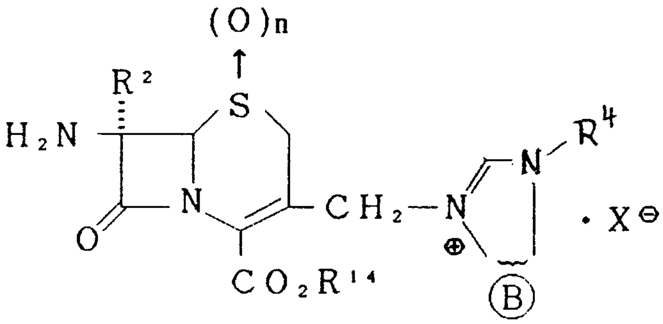

+ $R^1COOH$

(V)

(VI)

or a reactive derivative in amino group, or salt thereof

or a reactive derivative in carboxyl group, or salt thereof

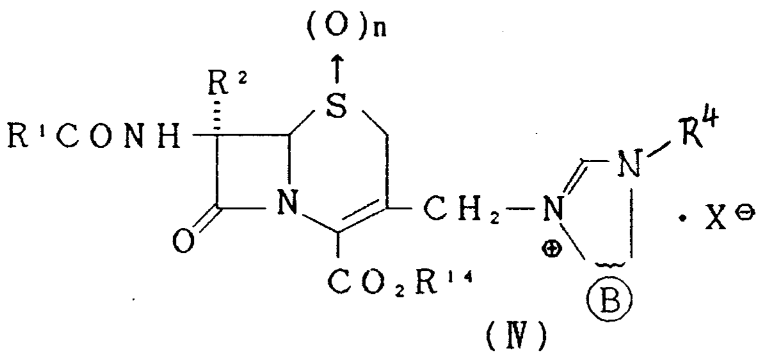

(IV)

or salt thereof

wherein $R^1$, $R^2$, $R^4$, $R^{14}$, B, $X^{\ominus}$ and n are same as above.

Compound (IV) or salt thereof can be obtained by reacting Compound (V), a reactive derivative in amino group or salt thereof and Compound (VI), a reactive derivative in carboxyl group or salt thereof. As salts of Compounds (V) and (VI) are used the same salts enumerated with respect to Compound (I). Examples of reactive derivatives of Compound (V) in amino group are same compounds which are mentioned with respect to Compound (VII) in Synthetic route-1.

As reactive derivatives of Compound (VI) in carboxyl group are enumerated the same compounds as above.

In the reaction are used the same solvents as described in the above Synthetic route-1. The reaction temperature is not particularly limited and the reaction can be conducted under cooling or heating and preferably at about -20 to +40 °C.

The reaction can be conducted in the presence of an organic or inorganic base. As the bases are used the same compounds as mentioned in the above Synthetic route-1. The amounts of the base to be used are not particularly limited but are usually up to 25 equivalents, preferably 0.25 to 4 equivalents per equivalent of Compound (V).

In the above reaction, the proportions of Compound (V) and Compound (VI) to be used are not particularly limited and are selected from a wide range. However, it is preferable to use in the equivalent ratio of the former : the latter of 1:5 to 5:1, more preferably of 1:2 to 2:1.

In case Compound (VI) is used in the form of free acid or salt thereof, the reaction is conducted preferably in the presence of a condensing agent. As the condensing agents are employed the same compounds as described in the above Synthetic route-1. The above condensing agent is used usually in an amount of up to 25 equivalents, preferably 0.25 to 4 equivalents per equivalent of Compound (V).

When $R^1$ in Compound (IV) is amino-protecting group or carboxyl-protecting group, or when n is 1, Compound (I) is obtained by removing amino- or carboxyl-protecting group, or by the reduction, respectively. As the elimination reaction or reduction is conducted the same reaction as mentioned in the above Synthetic route-1.

The present Compound (I) or (I') has an excellent anti-bacterial activity and is useful as an agent for preventing and treating bacterial infections.

For preventing and treating is used a usual preparation containing, as an effective component, the present Compound (I) or (I') or a salt thereof and a pharmaceutically acceptable carrier such as an organic, inorganic, solid or liquid adjuvant suitable to oral, parenteral or local administration. The preparation may be in the form of a tablet, granule, powder, capsule and like solid form, or solution, suspension, syrup, emulsion, lemonade and like liquid form. To the preparation is added as required an auxiliary substance, stabilizer, lubricant, and other usual additives such as lactose, magnesium stearate, kaolin, sucrose, corn starch, talc, stearic acid, gelatin, agar, pectin, peanut oil, olive oil, cacao butter, ethylene glycol, etc.

The dosage of Compound (I ) or (I') varies depending on age and situation of a patient, kind of disease or compound to be administered, etc. Usually, about 1 to 4000mg or more of Compound (I) or (I') can be daily administered to a patient. For treating bacterial infections is administered Compound (I) or (I') in an average dosage per one time of about 50mg, 100mg, 250mg, 500mg, 1000mg or 2000mg.

The invention will be described with reference to Examples and Test Example. In the following, Tr is trityl group, tBu tertiary butyl group.

Example 1

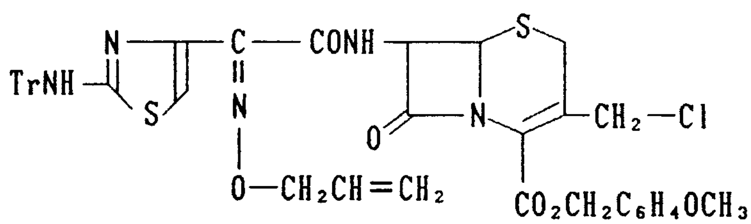

Triethylamine (0.19ml) was added with stirring to a suspension of p-toluenesulfonic acid salt of 7-amino-3-chloromethyl-3-cephem-4-carboxylic acid p-methoxybenzyl ester (700mg) in methylene chloride (20ml) under a condition of ice-cooling. To the mixture was added (Z)-2-(2-allyloxyimino)-2-(2-tritylaminothiazole-4-yl)acetic acid (600mg) and stirred to obtain a homogeneous solution. To the solution were added with stirring and under a condition of ice-cooling, 1-hydroxybenzotriazole (265mg) and then dicyclohexylcarbodiimide (270mg). The mixture was further stirred for 3 hours under ice-cooling. The mixture was filtered and the resulting white solid was washed with a small amount of acetone. The filtrate and washing liquid were combined and concentrated to remove a solvent at a reduced pressure. The residue was eluted through silicagel column with use of chloroform-methanol (volume ratio 8:1). The eluted solution containing a desired product was collected and concentrated to obtain 7-[2-(2-propenyloxyimino)-2-(2-tritylaminothiazole-4-yl)acetamide]-3-chloromethyl-3-cephem-4-carboxylic acid p-methoxybenzyl ester (syn-isomer) (900mg).

NMR δ ppm ($CDCl_3$):

3.50(2H,s), 3.78(3H,s), 4.45(2H,d,J = 12Hz), 4.58(2H,d,J = 5.5Hz), 4.96(1H,d,J = 5.0Hz), 5.1~5.4(4H,m), 5.8~6.1(2H,m), 6.7(1H,s), 7.08~7.84(20H,m), 8.13(1H,s)

Example 2

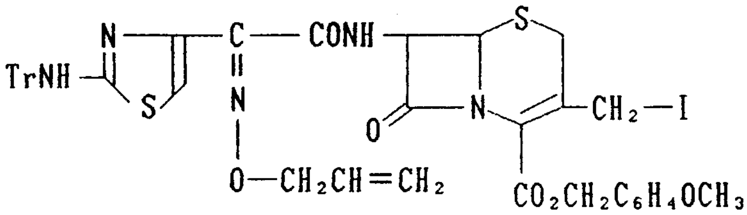

To a solution of 890mg of 7-[2-(2-propenyloxyimino)-2-(2-tritylaminothiazole-4-yl)acetamide]-3-chloromethyl-3-cephem-4-carboxylic acid p-methoxybenzyl ester (syn-isomer) in 50ml of acetone were added 237mg of potassium iodide and 157mg of sodium carbonate. The mixture was heated to reflux for one hour with stirring. The mixture was allowed to cool to room temperature and filtered. The filtrate was concentrated at a reduced pressure. The residue was dissolved in 50ml of methylene chloride and was washed with 30ml of 5% aqueous solution of sodium thiosulfate. The resulting organic layer was concentrated at a reduced pressure to obtain 820mg of 7-[2-(2-propenyloxyimino)-2-(2-tritylaminothiazole-4-yl)acetamide]-3-iodomethyl-3-cephem-4-carboxylic acid p-methoxybenzyl ester (syn-isomer).

NMR $\delta$ ppm ($CDCl_3$):

3.50(2H,m), 3.82(3H,s), 4.32(2H,s), 4.70(2H,d,J = 5.5Hz), 4.90(1H,d,J = 5.0Hz), 5.1~5.4(4H,m), 5.8~6.1(2H,m)-,6.7(1H,s), 7.08~7.84(20H,m), 8.13(1H,s)

Example 3

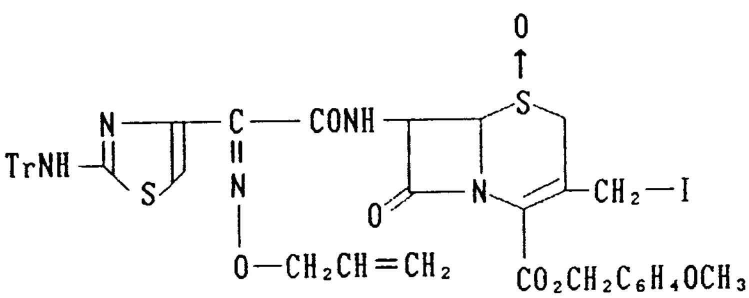

To a solution cooled at 0°C of 5.0g of 7-[2-(2-propenyloxyimino)-2-(2-tritylaminothiazole-4-yl)-acetamide]-3-iodomethyl-3-cephem-4-carboxylic acid p-methoxybenzyl ester (syn-isomer) in 160ml of methylene chloride was added dropwise a solution of 1.5g of m-chloroperbenzoic acid in 40ml of methylene chloride in 10 minutes and the mixture was stirred for 50 minutes at the same temperature. The mixture was allowed to cool to room temperature and was washed with an aqueous solution of sodium carbonate, water and saturated aqueous solution of NaCl. After dehydrated with use of magnesium sulfate, the organic layer was concentrated at a reduced pressure to remove a solvent, giving 5.1g of 7-[2-(2-propenyloxyimino)-2-(2-tritylaminothiazole-4-yl)acetamide]-3-iodomethyl-3-cephem-1-oxido-4-carboxylic acid p-methoxybenzyl ester (syn-isomer).

NMR $\delta$ ppm ($CDCl_3$):

3.54(2H,m,), 3.78(3H,s), 4.41(2H,m), 4.44(1H,m), 4.73(2H,m), 4.97~5.42(2H,m), 5.22(2H,s), 5.65~6.21(2H,m), 6.64(1H,s), 7.08(4H,m), 7.25(16H,s)

Example 4

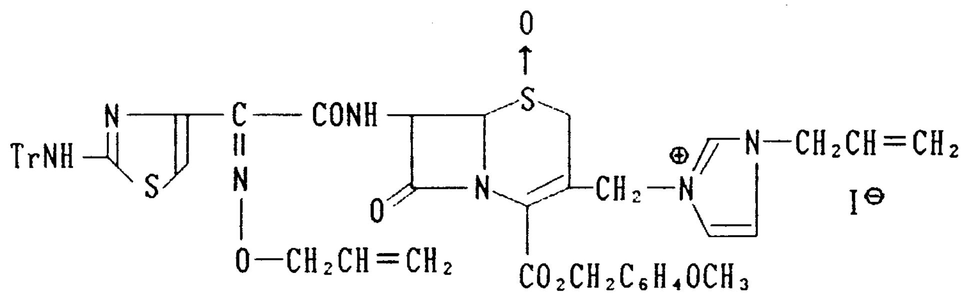

Into a mixture of THF (5ml) and chloroform (2.5ml) was dissolved 0.5g of 7-[2-(2-propenyloxyimino)-2-(2-tritylaminothiazole-4-yl)acetamide]-3-iodomethyl-3-cephem-1-oxido-4-carboxylic acid p-methoxybenzyl ester (syn-isomer). To the solution was added with ice-cooling 64.8mg of 1-(2-propenyl)imidazole. After the addition, the mixture was heated to room temperature and was further stirred for 3 hours. Solvents were removed at a reduced pressure. The residue was wade into a powder with use of diethyl ether and purified by a silicagel column (chloroform/methanol = 8/1, volume ratio) to collect eluate containing a desired compound. By removing a solvent was obtained 375.1mg of 7-[2-(2-propenyloxyimino)-2-(2-tritylaminothiazole-4-yl)acetamide]-3-[3-(2-propenyl)-1-imidazoliomethyl]-3-cephem-1-oxido-4-carboxylic acid p-methoxybenzyl ester•iodide (syn-isomer).
NMR δ ppm (DMSO-$d_6$):
3.75(2H,m), 3.80(3H,s), 4.60(2H,m), 4.88~5.47(9H,m), 5.60~6.30(3H,m), 6.81(1H,s), 7.14(4H,m), 7.32(15H,s), 7.66(2H,m), 8.74(1H,s), 8.94(1H,m), 9.06(1H,s)

Example 5

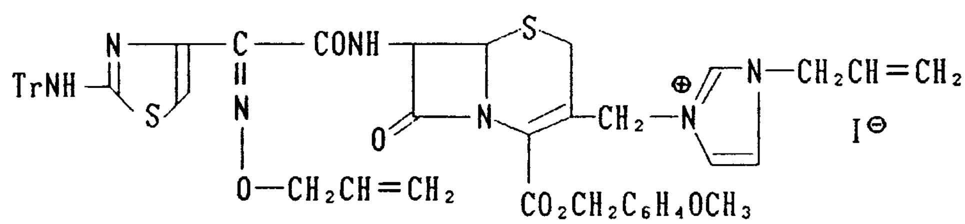

Into 20ml of acetone was dissolved 359.6mg of 7-[2-(2-propenyloxyimino)-2-(2-tritylaminothiazole-4-yl)-acetamide]-3-[3-(2-propenyl)-1-imidazoliomethyl]-3-cephem-1-oxido-4-carboxylic acid p-methoxybenzyl ester•iodide (syn-isomer). The solution was cooled to -40° C with dry ice-acetone refrigerant and added dropwise 270μl of phosphorus tribromide thereto. The mixture was stirred for one hour. With cooling at the same temperature, 3ml of aqueous solution of 500mg of sodium carbonate was added to the mixture. After heated to room temperature, 60ml of water was added and the mixture was extracted twice with 100ml of ethyl acetate. The resulting organic layer was dried with anhydrous magnesium sulfate. By removing ethyl acetate was obtained 292.8mg of 7-[2-(2-propenyloxyimino)-2-(2-tritylaminothiazole-4-yl)acetamide)-3-[3-(2-propenyl)-1-imidazoliomethyl]-3-cephem-4-carboxylic acid p-methoxybenzyl ester•iodide (syn-isomer).
NMR δ ppm (DMSO-$d_6$):
3.50(2H,s), 3.78(3H,s), 4.56(2H,m), 4.84(2H,m), 5.01~5.60(9H,m), 5.60~6.40(3H,m), 6.72(1H,s), 7.15(4H,m), 7.31(15H,s), 7.62(2H,m), 9.05(1H,s), 9.20(1H,s), 9.53(1H,m)

Example 6

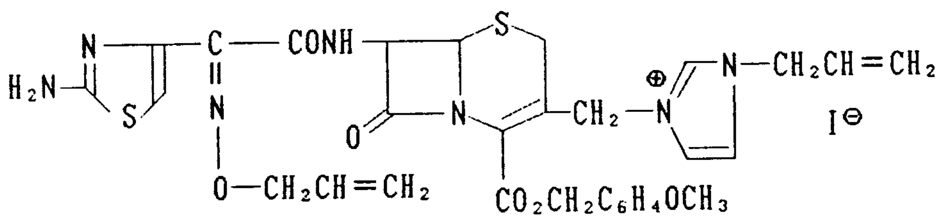

Into 3ml of 80% aqueous solution of acetic acid was dissolved 280mg of 7-[2-(2-propenyloxyimino)-2-(2-tritylaminothiazole-4-yl)acetamide]-3-[3-(2-propenyl)-1-imidazoliomethyl]-3-cephem-4-carboxylic acid p-methoxybenzyl ester•iodide (syn-isomer). The solution was stirred at 35 to 40°C for 2 hours and the solvent was removed by freeze-drying. To the residue was added diethyl ether and powder separated was collected by filtration to obtain 209.2mg of 7-[2-(2-aminothiazole-4-yl)-2-(2-propenyloxyimino)acetamide]-3-[3-(2-propenyl)-1-imidazoliomethyl]-3-cephem-4-carboxylic acid p-methoxybenzyl ester•iodide (syn-isomer).
NMR δ ppm (DMSO-$d_6$):
3.54(2H,s), 3.76(3H,s), 4.58(2H,m), 4.83(2H,m), 5.0~5.54(9H,m), 5.61~6.35(3H,m), 6.78(1H,s), 6.83(2H,s), 7.13(4H,m), 7.68(2H,m), 9.22(1H,s), 9.63(1H,s)

Example 7

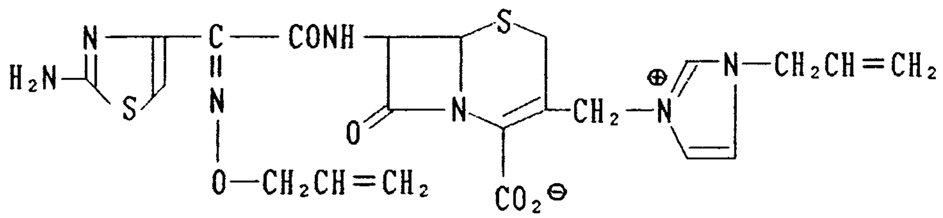

In a mixture of methylene chloride (3ml), trifluoroacetic acid (930μl) and anisole (650μl) was added 190mg of 7-[2-(2-aminothiazole-4-yl)-2-(2-propenyloxyimino)acetamide]-3-[3-(2-propenyl)-1-imidazoliomethyl]-3-cephem-4-carboxylic acid p-methoxybenzyl ester•iodide (syn-isomer). The mixture was stirred at ice-cooling for 2 hours. The solvent was removed at a reduced pressure. To the residue was added 100ml of diethyl ether to separate out powder. The powder was collected by filtration, neutralized with an aqueous solution of potassium hydrogen carbonate and freeze-dried to obtain yellow crude powder. The powder was subjected to Sephadex LH-20 column (methanol) to obtain an eluate containing a desired compound. By removing a solvent at a reduced pressure was obtained 68.4mg of 7-[2-(2-aminothiazole-4-yl)-2-(2-propenyloxyimino)acetamide]-3-[3-(2-propenyl)-1-imidazoliomethyl]-3-cephem-4-carboxylate.
NMR δ ppm (DMSO-$d_6$):
3.65(2H,m), 4.57(2H,m), 4.82(2H,m), 5.01~5.5(7H,m), 5.55~6.35(3H,m), 6.83(1H,s), 7.17(2H,s), 7.63(2H,m), 9.23(1H,s), 9.35(1H,d,J=8.0Hz)

Example 8

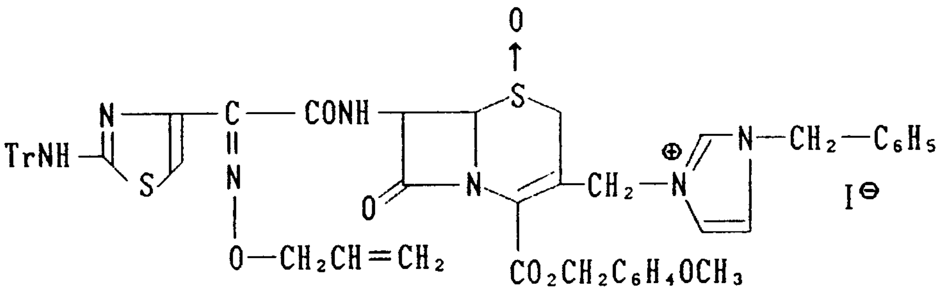

In the same manner as in Example 4, from 0.5g of 7-[2-(2-propenyloxyimino)-2-(2-tritylaminothiazole-4-yl)acetamide]-3-iodomethyl-3-cephem-1-oxido-4-carboxylic acid p-methoxybenzyl ester (syn-isomer) and 94.8mg of 1-benzylimidazole was obtained 418.3mg of 7-[2-(2-propenyloxyimino)-2-(2-tritylaminothiazole-4-yl)acetamide]-3-(3-benzyl-1-imidazoliomethyl)-3-cephem-1-oxido-4-carboxylic acid p-methoxybenzyl ester•iodide (syn-isomer).
NMR δ ppm (DMSO-$d_6$):
3.65(2H,m), 3.28(3H,s), 4.56(2H,m), 4.97(1H,m), 5.01~5.60(8H,m), 5.51~6.30(2H,m), 6.80(1H,s), 7.12(4H,m), 7.32(15H,s), 7.43(5H,s), 7.62(1H,s), 7.73(1H,s), 8.73(1H,s), 8.91(1H,m), 9.40(1H,s)

Example 9

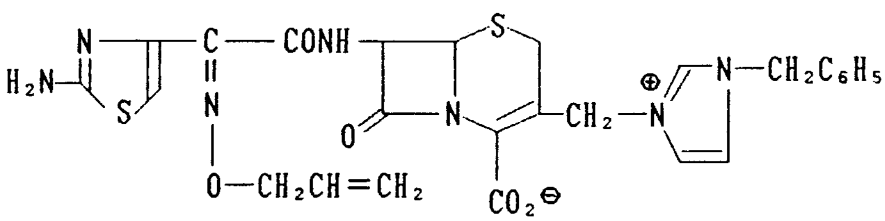

In the same manner as in Examples 5, 6 and 7, from 408.3mg of 7-[2-(2-propenyloxyimino)-2-(2-tritylaminothiazole-4-yl)acetamide]-3-(3-benzyl-1-imidazoliomethyl)-3-cephem-1-oxido-4-carboxylic acid p-methoxybenzyl ester•iodide (syn-isomer) was obtained 37.2mg of 7-[2-(2-aminothiazole-4-yl)-2-(2-propenyloxyimino)acetamide]-3-(3-benzyl-1-imidazoliomethyl)-3-cephem-4-carboxylate (syn-isomer).
NMR δ ppm (DMSO-$d_6$):
3.47(2H,m), 4.68(2H,m), 4.80~5.50(5H,m), 5.41(2H,s), 5.60~6.23(2H,m), 6.93(1H,s), 7.37(5H,s), 7.67(2H,m), 9.23(1H,s)

Example 10

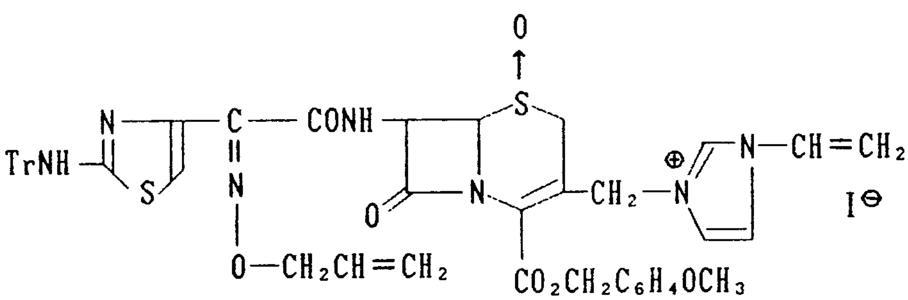

In the same manner as in Example 4, from 0.5g of 7-[2-(2-propenyloxyimino)-2-(2-tritylaminothiazole-4-yl)acetamide]-3-iodomethyl-3-cephem-1-oxido-4-carboxylic acid p-methoxybenzyl ester (syn-isomer) and 57µl of 1-vinylimidazole was obtained 380mg of 7-[2-(2-propenyloxyimino)-2-(2-tritylaminothiazole-4-yl)-acetamide]-3-(3-vinyl-1-imidazoliomethyl)-3-cephem-1-oxido-4-carboxylic acid p-methoxybenzyl ester•iodide (syn-isomer).

NMR δ ppm (DMSO-$d_6$):

3.75(2H,m), 3.80(3H,s), 4.60(2H,m), 4.85~5.47(9H,m), 7.32(15H,s), 7.66(2H,m), 8.74(1H,s), 8.94(1H,m), 9.06-(1H,s)

Example 11

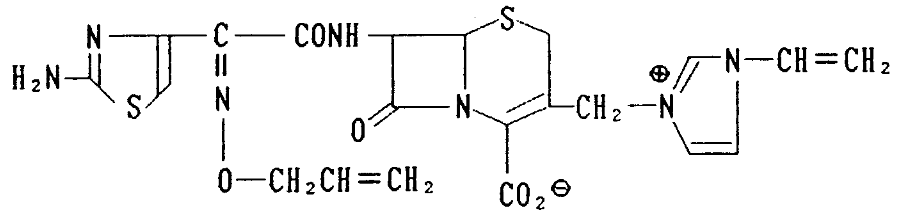

In the same manner as in Examples 5, 6 and 7, from 350mg of 7-[2-(2-propenyloxyimino)-2-(2-tritylaminothiazole-4-yl)acetamide]-3-(3-vinyl-1-imidazoliomethyl)-3-cephem-1-oxido-4-carboxylic acid p-methoxybenzyl ester•iodide (syn-isomer) was obtained 29mg of 7-[2-(2-aminothiazole-4-yl)-2-(2-propenyloxyimino)acetamide]-3-(3-vinyl-1-imidazoliomethyl) -3-cephem-4-carboxylate (syn-isomer).

NMR δ ppm (DMSO-$d_6$):

3.75(2H,m), 4.60(2H,m), 4.80~5.47(9H,m), 6.78(1H,s), 6.83(2H,s), 7.17(2H,s), 9.23(1H,s), 9.35-(1H,d,J = 8.0Hz)

Example 12

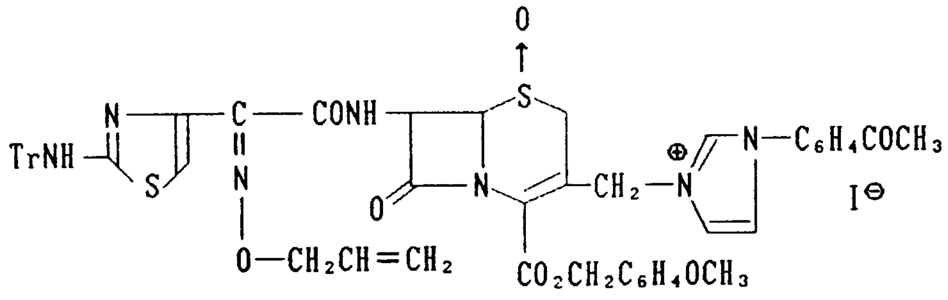

In the same manner as in Example 4, from 0.5g of 7-[2-(2-propenyloxyimino)-2-(2-tritylaminothiazole-4-yl)acetamide]-3-iodomethyl-3-cephem-1-oxido-4-carboxylic acid p-methoxybenzyl ester (syn-isomer) and 100µl of 4-imidazole acetophenone was obtained 330mg of 7-[2-(2-propenyloxyimino)-2-(2-tritylaminothiazole-4-yl)acetamide]-3-(3-acetophenyl-1-imidazoliomethyl)-3-cephem-1-oxido-4-carboxylic acid p-methoxybenzyl ester•iodide (syn-isomer).

NMR δ ppm (DMSO-$d_6$):

3.80(3H,s), 3.75(2H,m), 4.50(2H,m), 4.56(2H,m), 5.0(2H,m), 5.15(4H,m), 6.60(1H,s), 7.32(18H,m), 9.30-(1H,d,J = 8.0Hz)

Example 13

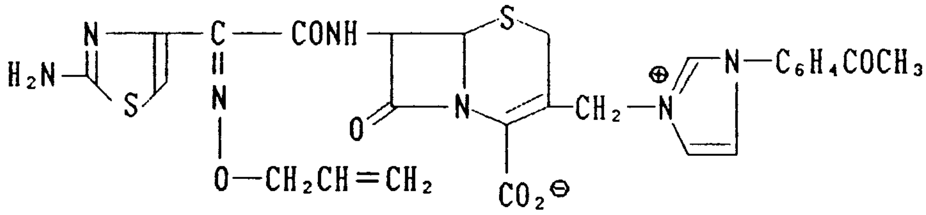

In the same manner as in Examples 5, 6 and 7, from 310mg of 7-[2-(2-propenyloxyimino)-2-(2-tritylaminothiazole-4-yl)acetamide]-3-(3-acetophenyl-1-imidazoliomethyl)-3-cephem-1-oxido-4-carboxylic acid p-methoxybenzyl ester•iodide (syn-isomer) was obtained 53mg of 7-[2-(2-aminothiazole-4-yl)-2-(2-propenyloxyimino)acetamide]-3-(3-acetophenyl-1-imidazoliomethyl)-3-cephem-4-carboxylate.
NMR δ ppm (DMSO-$d_6$):
3.75(2H,m), 4.50(2H,m), 5.0(2H,m), 5.50(1H,m), 6.65(1H,s), 7.0(2H,s), 9.06(1H,d,J = 8.0Hz)

Example 14

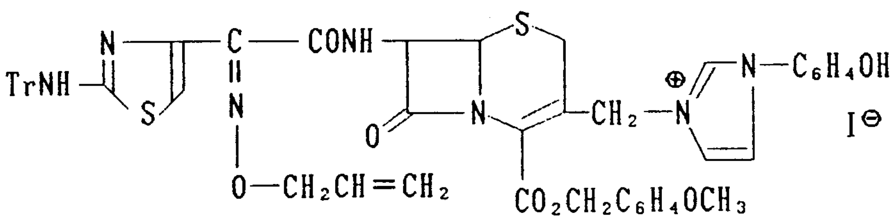

In the same manner as in Example 4, from 0.5g of 7-[2-(2-propenyloxyimino)-2-(2-tritylaminothiazole-4-yl)acetamide]-3-iodomethyl-3-cephem-1-oxido-4-carboxylic acid p-methoxybenzyl ester (syn-isomer) and 88μl of 4-imidazole acetophenone was obtained 310mg of 7-[2-(2-propenyloxyimino)-2-(2-tritylaminothiazole-4-yl)acetamide)-3-[3-(4-hydroxyphenyl)-1-imidazoliomethyl)-3-cephem-1-oxido-4-carboxylic acid p-methoxybenzyl ester•iodide (syn-isomer).
NMR δ ppm (DMSO-$d_6$):
3.80(3H,s), 3.75(2H,m), 4.50(2H,m), 4.56(2H,m), 5.75~6.10(4H,m), 7.0~7.32(17H,m), 9.20(1H,d,J = 8.0Hz)

Example 15

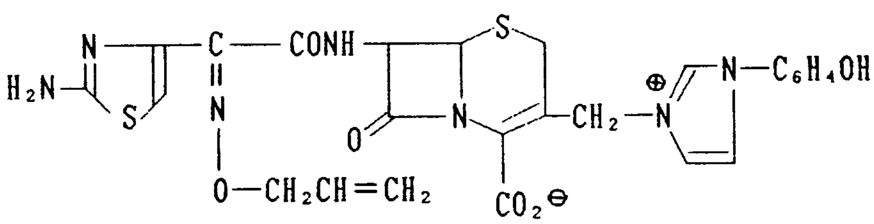

In the same manner as in Examples 5, 6 and 7, from 290mg of 7-[2-(2-propenyloxyimino)-2-(2-tritylaminothiazole-4-yl)acetamide]-3-[3-(4-hydroxyphenyl)-1-imidazoliomethyl]-3-cephem-1-oxido-4-carboxylic acid p-methoxybenzyl ester•iodide (syn-isomer) was obtained 53mg of 7-[2-(2-aminothiazole-4-yl)-2-(2-propenyloxyimino)acetamide]-3-[3-(4-hydroxyphenyl)-1-imidazoliomethyl]-3-cephem-4-carboxylate (syn-isomer).
NMR δ ppm (DMSO-$d_6$):

3.75(2H,m), 4.50(2H,m), 4.56(2H,m), 5.75~6.10(4H,m), 7.2(6H,m), 9.20(1H,d,J = 8.0Hz)

Example 16

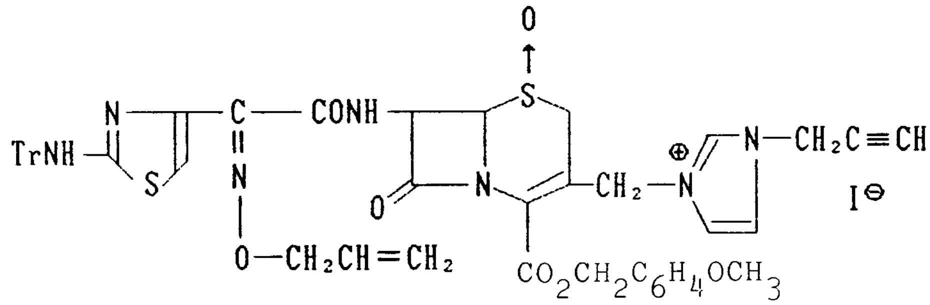

In the same wanner as in Example 4, from 0.5g of 7-[2-(2-propenyloxyimino)-2-(2-tritylaminothiazole-4-yl)acetamide]-3-iodomethyl-3-cephem-1-oxido-4-carboxylic acid p-methoxybenzyl ester (syn-isomer) and 58μl of 1-propynylimidazole was obtained 325mg of 7-[2-(2-propenyloxyimino)-2-(2-tritylaminothiazole-4-yl)-acetamide]-3-[3-(2-propynyl)-1-imidazoliomethyl]-3-cephem-1-oxido-4-carboxylic acid p-methoxybenzyl ester•iodide (syn-isomer).
NMR δ ppm (DMSO-$d_6$):
3.9~4.4(2H,m), 4.6(2H,s), 4.85(1H,d,J = 5.0Hz), 5.45(1H,m), 6.72(1H,s), 7.25(15H,s), 9.45(1H,d,J = 8.0Hz)

Example 17

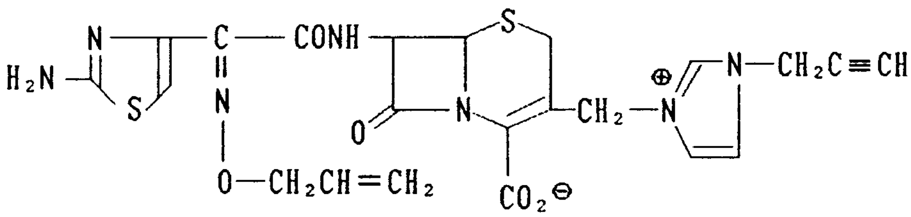

In the same manner as in Examples 5, 6 and 7, from 315mg of 7-[2-(2-propenyloxyimino)-2-(2-tritylaminothiazole-4-yl)acetamide]-3-[3-(2-propynyl)-1-imidazoliomethyl]-3-cephem-1-oxido-4-carboxylic acid p-methoxybenzyl ester•iodide (syn-isomer) was obtained 38mg of 7-[2-(2-aminothiazole-4-yl)-2-(2-propenyloxyimino)acetamide)-3-[3-(2-propynyl)-1-imidazoliomethyl]-3-cephem-4-carboxylate.
NMR δ ppm (DMSO-$d_6$):
3.9~4.4(2H,m), 4.6(2H,s), 4.85(1H,d,J = 5.0Hz), 5.45(1H,m), 6.72(1H,s), 7.10(2H,s), 9.45(1H,d,J = 8.0Hz)

Example 18

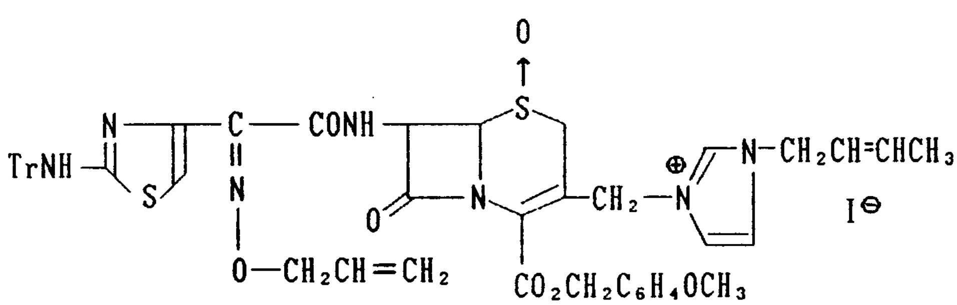

In the same manner as in Example 4, from 0.5g of 7-[2-(2-propenyloxyimino)-2-(2-tritylaminothiazole-4-yl)acetamide]-3-iodomethyl-3-cephem-1-oxido-4-carboxylic acid p-methoxybenzyl ester (syn-isomer) and 68μl of 1-(2-butenyl)imidazole was obtained 365mg of 7-[2-(2-propenyloxyimino)-2-(2-tritylaminothiazole-4-yl)acetamide]-3-[3-(2-butenyl)-1-imidazoliomethyl]-3-cephem-1-oxido-4-carboxylic acid p-methoxybenzyl ester•iodide (syn-isomer).
NMR δ ppm (DMSO-$d_6$):
1.7(3H,d,J=6.0), 3.9~4.4(2H,m), 4.6(2H,s), 4.85(1H,d,J=5.0Hz), 5.45(1H,m), 6.72(1H,s), 7.25(15H,s), 9.45-(1H,d,J=8.0Hz)

Example 19

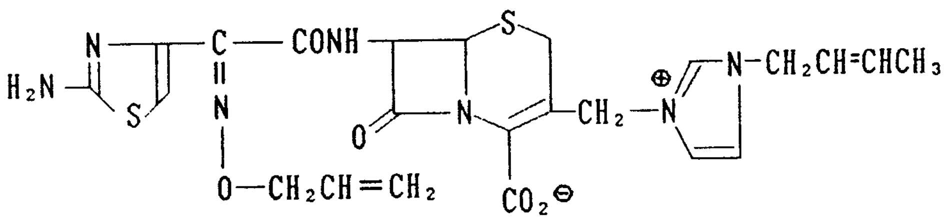

In the same manner as in Examples 5, 6 and 7, from 353mg of 7-[2-(2-propenyloxyimino)-2-(2-tritylaminothiazole-4-yl)acetamide]-3-[3-(2-butenyl)-1-imidazoliomethyl]-3-cephem-1-oxido-4-carboxylic acid p-methoxybenzyl ester•iodide was obtained 57mg of 7-[2-(2-aminothiazole-4-yl)-2-(2-propenyloxyimino)-acetamide]-3-[3-(2-butenyl)-1-imidazoliomethyl]-3-cephem-4-carboxylate (syn-isomer).
NMR δ ppm (DMSO-$d_6$):
1.7(3H,d,J=6.0), 3.9~4.4(2H,m), 4.6(2H,s), 4.85(1H,d,J=5.0Hz), 5.45(1H,m), 6.73(1H,s), 7.10(2H,s), 9.45-(1H,d,J=8.0Hz)

Example 20

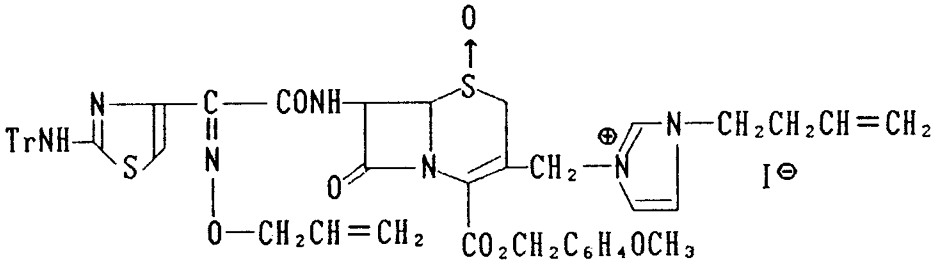

In the same manner as in Example 4, from 0.5g of 7-[2-(2-propenyloxyimino)-2-(2-tritylaminothiazole-4-yl)acetamide]-3-iodomethyl-3-cephem-4-carboxylic acid p-methoxybenzyl ester (syn-isomer) and 68μl of 1-(3-butenyl)imidazole was obtained 353mg of 7-[2-(2-propenyloxyimino)-2-(2-tritylaminothiazole-4-yl)-acetamide]-3-[3-(3-butenyl)-1-imidazoliomethyl)-3-cephem-1-oxido-4-carboxylic acid p-methoxy benzyl ester•iodide (syn-isomer).
NMR δ ppm (DMSO-$d_6$):
2.28(2H,m), 3.85(2H,m), 4.6(2H,s), 4.85(1H,d,J=5.0Hz), 5.45(1H,m), 6.76(1H,s), 7.25(15H,s), 9.20-(1H,d,J=8.0Hz)

Example 21

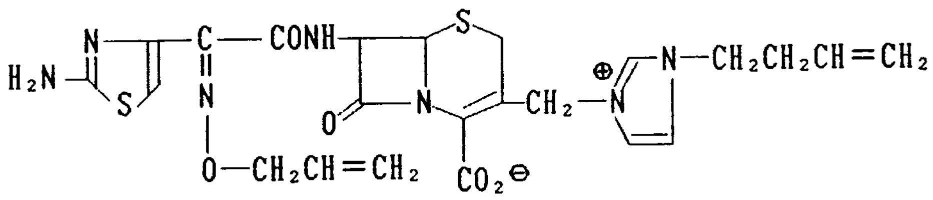

In the same manner as in Examples 5, 6 and 7, from 348mg of 7-[2-(2-propenyloxyimino)-2-(2-tritylaminothiazole-4-yl)acetamide]-3-[3-(3-butenyl)-1-imidazoliomethyl]-3-cephem-1-oxido-4-carboxylic acid p-methoxybenzyl ester•iodide (syn-isomer) was obtained 51mg of 7-[2-(2-aminothiazole-4-yl)-2-(2-propenyloxyimino)acetamide]-3-[3-(3-butenyl)-1-imidazoliomethyl]-3-cephem-4-carboxylate (syn-isomer).
NMR δ ppm (DMSO-$d_6$):
2.28(2H,m), 3.85(2H,m), 4.6(2H,s), 4.85(1H,d,J = 5.0Hz), 5.45(1H,m), 6.76(1H,s), 7.01(2H,s), 9.20-(1H,d,J = 8.0Hz)

Example 22

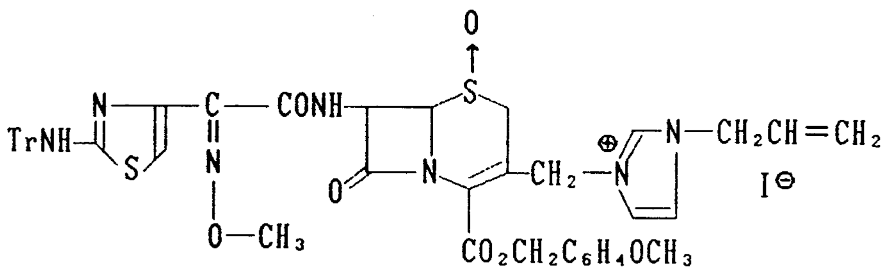

In the same manner as in Example 4, from 0.6g of 7-[2-methoxyimino-2-(2-tritylaminothiazole-4-yl)-acetamide]-3-iodomethyl-3-cephem-1-oxido-4-carboxylic acid p-methoxybenzyl ester (syn-isomer) and 79mg of 1-(2-propenyl)imidazole was obtained 390mg of 7-[2-methoxyimino-2-(2-tritylaminothiazole-4-yl)-acetamide]-3-[3-(2-propenyl)-1-imidazoliomethyl]-3-cephem-1-oxido-4-carboxylic acid p-methoxybenzyl ester•iodide (syn-isomer).
NMR δ ppm ($CDCl_3$):
3.77(3H,s), 4.0(3H,s), 4.95(1H,d,J = 4Hz), 5.6~6.5(2H,m), 6.65(1H,s), 6.7~7.5(19H,m), 7.7(1H,s), 9.7(1H,s)

Example 23

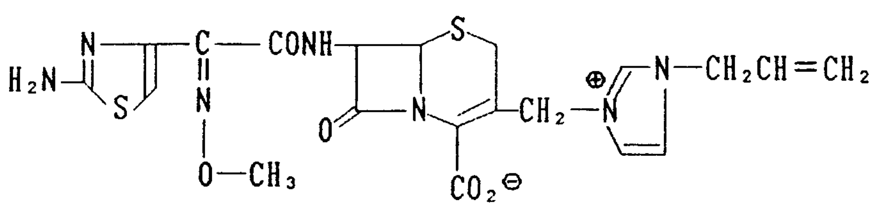

In the same manner as in Examples 5, 6 and 7, from 320mg of 7-[2-methoxyimino-2-(2-tritylaminothiazole-4-yl)acetamide]-3-[3-(2 -propenyl) -1-imidazoliomethyl]-3-cephem-1-oxido-4-carboxylic acid p-methoxybenzyl ester•iodide (syn-isomer) was obtained 26mg of 7-[2-(2-aminothiazole-4-yl)-2-methoxyiminoacetamide]-3-[3-(2-propenyl)-1-imidazoliomethyl]-3-cephem-4-carboxylate (syn-isomer).
NMR δ ppm (DMSO-$d_6$):

3.80(3H,s), 4.48~6.25(m8H), 6.65(1H,s), 7.1(2H,s), 7.6(1H,s), 7.95(1H,s), 9.25(1H,s), 9.4(1H,d,J=8.0Hz)

Example 24

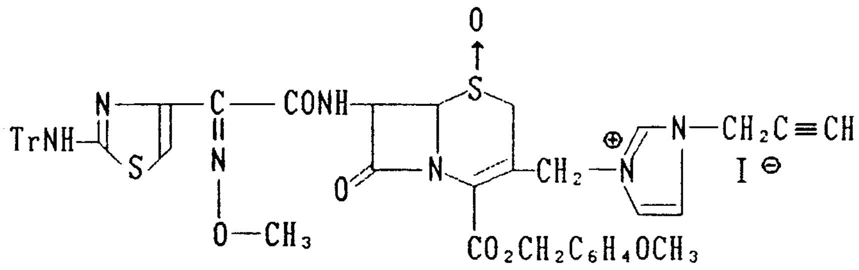

In the same manner as in Example 4, from 0.6g of 7-[2-methoxyimino-2-(2-tritylaminothiazole-4-yl)-acetamide]-3-iodomethyl-3-cephem-1-oxido-4-carboxylic acid p-methoxybenzyl ester (syn-isomer) and 88mg of 1-propynylimidazole was obtained 330mg of 7-[2-methoxyimino-2-(2-tritylaminothiazole-4-yl)-acetamide]-3-[3-(2-propynyl)-1-imidazoliomethyl)-3-cephem-1-oxido-4-carboxylic acid p-methoxybenzyl ester•iodide (syn-isomer).

NMR δ ppm ($CDCl_3$):

2.75(1H,m), 3.75(3H,s), 4.0(3H,s), 4.75(1H,d,J=3.0Hz), 4.8~5.5(6H,m), 6.0~6.3(1H,m), 6.70(1H,s), 6.8~7.75-(20H,m), 9.8(1H,s)

Example 25

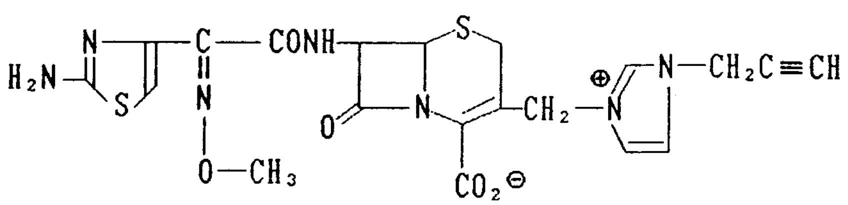

In the same manner as in Examples 5, 6 and 7, from 300mg of 7-[2-methoxyimino-2-(2-tritylaminothiazole-4-yl)acetamide]-3-[3-(2-propynyl)-1-imidazoliomethyl]-3-cephem-1-oxido-4-carboxylic acid p-methoxybenzyl ester•iodide (syn-isomer) was obtained 52mg of 7-[2-(2-aminothiazole-4-yl)-2-methoxyiminoacetamide]-3-[3-(2-propynyl)-1-imidazoliomethyl]-3-cephem-4-carboxylate (syn-isomer).

NMR δ ppm (DMSO-$d_6$):

3.80(3H,s), 4.75~5.25(5H,m), 5.5(1H,m), 6.65(1H,s), 7.1(2H,s), 7.65(1H,s), 7.95(1H,s), 9.35(1H,d,J=8.0Hz), 9.45(1H,s)

Example 26

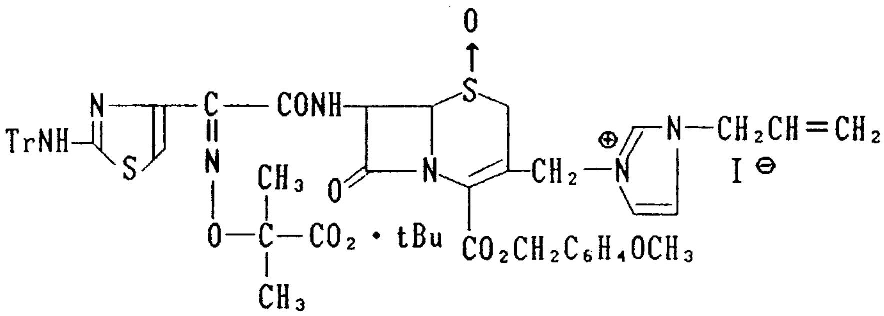

Into a mixture of THF (5ml) and chloroform (2.5ml) was dissolved 0.6g of 7-[2-(2-tert-butoxycarbonyl)-prop-2-oxyimino-2-(2-tritylaminothiazole-4-yl)acetamide]-3-iodomethyl-3-cephem-1-oxido-4-carboxylic acid p-methoxybenzyl ester (syn-isomer). To the solution which was cooled to 0° C was added 70mg of 1-(2-propenyl)imidazole. After the addition, the mixture was heated to room temperature and was further stirred for 3 hours. A solvent was removed at a reduced pressure. The residue was made into a powder with use of 50ml of diethyl ether and eluted by a silicagel column (chloroform/methanol = 8/1, volume ratio) to obtain 412.2mg of 7-[2-(2-tert-butoxycarbonyl)prop-2-oxyimino-2-(2-tritylaminothiazole-4-yl)-acetamide]-3-[3-(2-propenyl)-1-imidazoliomethyl]-3-cephem-1-oxido-4-carboxylic acid p-methoxybenzyl ester•iodide (syn-isomer).

NMR δ ppm (DMSO-$d_6$):

1.36(15H,s), 3.75(2H,m), 3.79(3H,s), 4.83(2H,m), 5.00(1H,m), 5.07~5.53(6H,m), 5.75~5.95(2H,m), 6.73(1H,s), 7.18(4H,m), 7.23(15H,s), 7.70(2H,m), 8.75(1H,s), 9.23(1H,m), 9.42(1H,s)

Example 27

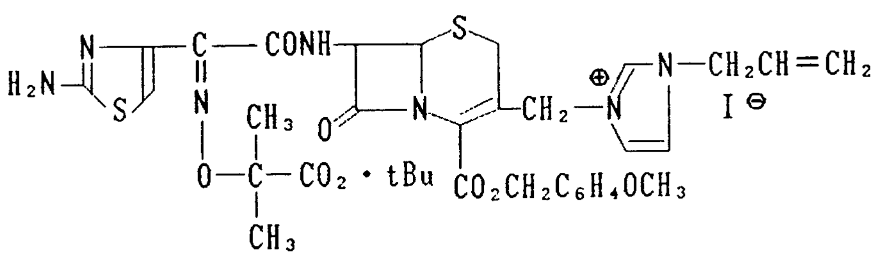

In the same manner as in Examples 5 and 6, from 400mg of 7-[2-(2-tert-butoxycarbonyl)prop-2-oxyimino-2-(2-tritylaminothiazole-4-yl)acetamide]-3-[3-(2-propenyl)-1-imidazoliomethyl)-3-cephem-1-oxido-4-carboxylic acid p-methoxybenzyl ester•iodide (syn-isomer) was obtained 230mg of 7-[2-(2-aminothiazole-4-yl)-2-(2-tert-butoxycarbonyl)prop-2-oxyiminoacetamide]-3-[3-(2-propenyl)-1-imidazoliomethyl]-3-cephem-4-carboxylic acid p-methoxybenzyl ester•iodide (syn-isomer).

NMR δ ppm (DMSO-$d_6$):

1.37(9H,s), 1.49(3H,s), 1.52(3H,s), 3.50(2H,s), 3.76(3H,s), 4.82(2H,m), 4.90~5.52(7H,m), 5.72~6.31(2H,m), 6.69(1H,s), 7.13(4H,m), 7.22(2H,s), 7.66(2H,m), 9.21(1H,s), 9.30(1H,m)

Example 28

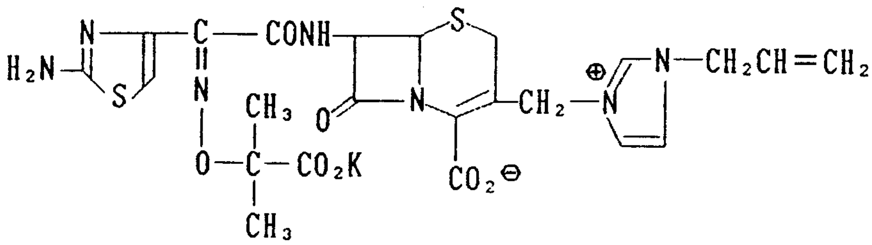

Into a mixture of methylene chloride (3ml) and anisole (2.2ml) was dissolved 220mg of 7-[2-(2-aminothiazole-4-yl)-2-(2-tert-butoxycarbonyl)prop-2-oxyiminoacetamide]-3-[3-(2-propenyl)-1-imidazoliomethyl]-3-cephem-4-carboxylic acid p-methoxybenzyl ester•iodide (syn-isomer). To the solution which was cooled to 0°C was added 3.1ml of trifluoroacetic acid. The mixture was stirred at 0°C for 2 hours and then at 10°C for 4 hours. A solvent was removed at a reduced pressure. The residue was made into a powder with use of 50ml of diethyl ether, collected by filtration, neutralized with an aqueous solution of potassium hydrogen carbonate and freeze-dried, giving yellow powder. The powder was eluted by use of Sephadex LH-20 (methanol) to obtain 53.2mg of white powder of 7-{2-(2-aminothiazole-4-yl)-2-[potassium-(2,2-dimethylacetate)oxyimino]acetamide}-3-[3-(2-propenyl)-1-imidazoliomethyl]-3-cephem-4-carboxylate (syn-isomer).

NMR $\delta$ ppm (DMSO-$d_6$):

1.38(3H,s), 1.43(3H,s), 3.50(2H,s), 4.83(2H,m), 4.91~5.60(5H,m), 5.75~6.37(2H,m), 6.68(1H,s), 7.07(2H,s), 7.58(1H,s), 7.48(1H,s), 9.46(1H,s), 12.13(1H,m)

Example 29

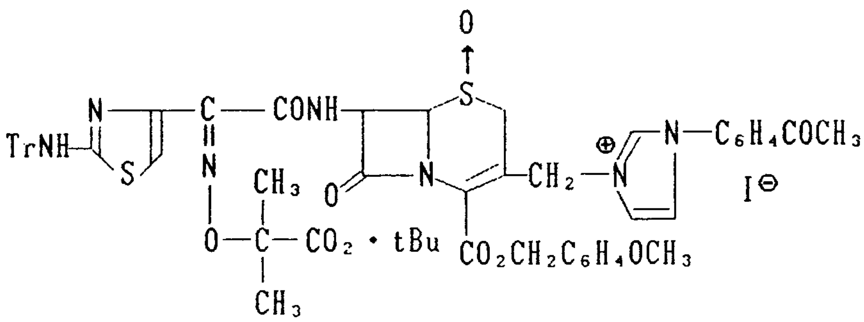

In the same manner as in Example 26, from 0.5g of 7-[2-(2-tert-butoxycarbonyl)prop-2-oxyimino-2-(2-tritylaminothiazole-4-yl)acetamide]-3-iodomethyl-3-cephem-1-oxido-4-carboxylic acid p-methoxybenzyl ester (syn-isomer) and 100µl of 4-imidazole acetophenone was obtained 340mg of 7-[2-(2-tert-butoxycarbonyl)-prop-2-oxyimino-2-(2-tritylaminothiazole-4-yl)acetamide]-3-(3-acetophenyl-1-imidazoliomethyl)-3-cephem-1-oxido-4-carboxylic acid p-methoxybenzyl ester•iodide (syn-isomer).

NMR $\delta$ ppm (DMSO-$d_6$):

1.36(15H,s), 3.75(2H,m), 3.60(3H,s), 4.83(2H,m), 6.73(1H,s), 7.18(4H,m), 7.25(15H,s), 9.42(1H,d,J=8.0Hz)

Example 30

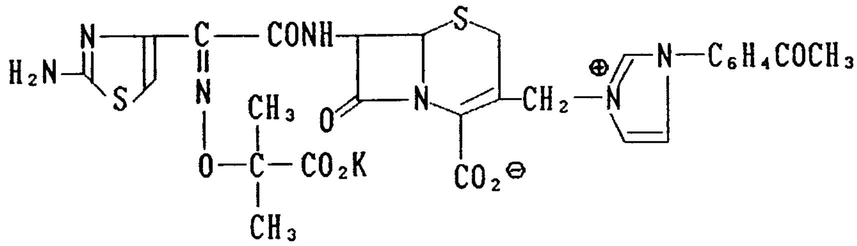

In the same manner as in Examples 27 and 28, from 330mg of 7-[2-(2-tert-butoxycarbonyl)prop-2-oxyimino-2-(2-tritylaminothiazole-4-yl)acetamide]-3-(3-acetophenyl-1-imidazoliomethyl)-3-cephem-1-oxido-4-carboxylic acid p-methoxybenzyl ester•iodide (syn-isomer) was obtained 53mg of 7-{2-(2-aminothiazole-4-yl)-2-[potassium(2,2-dimethylacetate)oxyimino]acetamide}-3-(3-acetophenyl-1-imidazoliomethyl)-3-cephem-4-carboxylate (syn-isomer).
NMR δ ppm (DMSO-$d_6$):
3.60(3H,s), 4.83(2H,m), 6.69(1H,s), 7.13(2H,s), 7.66(2H,m), 9.30(1H,d,J = 8.0Hz)

Example 31

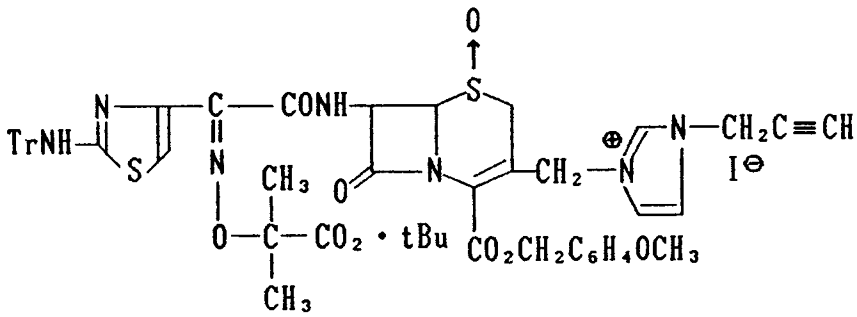

In the same manner as in Example 26, from 0.5g of 7-[2-(2-tert-butoxycarbonyl)prop-2-oxyimino-2-(2-tritylaminothiazole-4-yl)acetamide]-3-iodomethyl-3-cephem-1-oxido-4-carboxylic acid p-methoxybenzyl ester (syn-isomer) and 57mg of 1-(2-propynyl)imidazole was obtained 294mg of 7-[2-(2-tert-butoxycarbonyl)prop-2-oxyimino-2-(2-tritylaminothiazole-4-yl)-acetamide]-3-[3-(2-propynyl)-1-imidazoliomethyl]-3-cephem-1-oxido-4-carboxylic acid p-methoxybenzyl ester•iodide (syn-isomer).
NMR δ ppm ($CDCl_3$):
1.41(9H,s), 1.57(6H,s), 3.80(3H,s), 5.24(2H,s), 6.40(1H,m), 6.57(1H,s), 7.06(4H,m), 7.28(15H,s)

Example 32

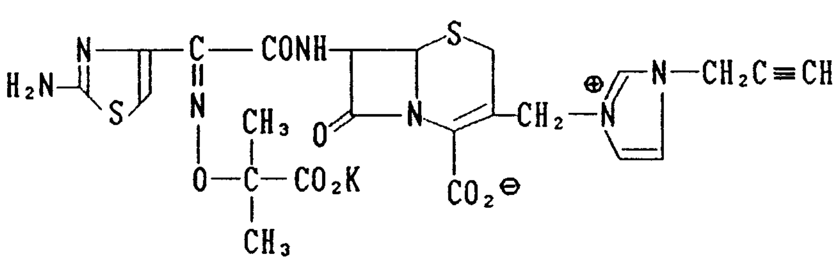

In the same manner as in Examples 27 and 28, from 290mg of 7-[2-(2-tert-butoxycarbonyl)prop-2-oxyimino-2-(2-tritylaminothiazole-4-yl)acetamide]-3-[3-(2-propynyl)-1-imidazoliomethyl]-3-cephem-1-oxido-4-carboxylic acid p-methoxybenzyl ester•iodide (syn-isomer) was obtained 34.2mg of 7-{2-(2-aminothiazole-4-yl)-2-[potassium(2,2-dimethylacetate) oxyimino]acetamide}-3-[3-(2-propynyl)-1-imidazoliomethyl]-3-cephem-4-carboxylate (syn-isomer).
NMR δ ppm (DMSO-$d_6$):
1.40(3H,s), 1.45(3H,s), 3.62(2H,s), 3.72(1H,m), 4.50~5.45(5H,m), 5.32(1H,m), 6.74(1H,s), 7.15(2H,s), 7.64-(1H,s), 7.96(1H,s), 9.15(1H,s), 11.88(1H,m)

Example 33

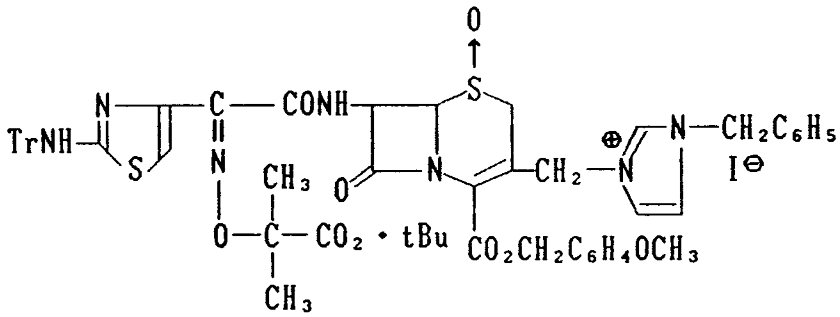

In the same manner as in Example 26, from 0.6g of 7-[2-(2-tert-butoxycarbonyl)prop-2-oxyimino-2-(2-tritylaminothiazole-4-yl)acetamide]-3-iodomethyl-3-cephem-1-oxido-4-carboxylic acid p-methoxybenzyl ester (syn-isomer) and 101mg of 1-benzylimidazole was obtained 409mg of 7-[2-(2-tert-butoxycarbonyl)prop-2-oxyimino-2-(2-tritylaminothiazole-4-yl)acetamide]-3-(3-benzyl-1-imidazoliomethyl)-3-cephem-1-oxido-4-carboxylic acid p-methoxybenzyl ester•iodide (syn-isomer).
NMR δ ppm (DMSO-$d_6$):
1.36(15H,s), 3.65(2H,m), 3.73(3H,s), 4.85~5.32(5H,m), 5.42(2H,s), 5.72(1H,m), 6.63(1H,s), 7.13(4H,m), 7.28-(15H,s), 7.42(5H,s), 7.71(2H,m), 8.76(1H,s), 9.23(1H,m, 9.32(1H,s)

Example 34

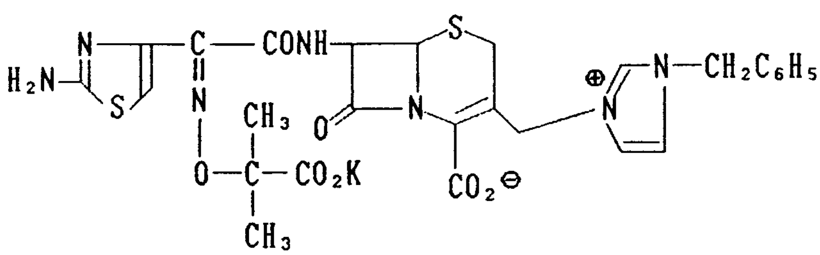

In the same manner as in Examples 27 and 28, from 480mg of 7-[2-(2-tert-butoxycarbonyl)prop-2-oxyimino-2-(2-tritylaminothiazole-4-yl)acetamide]-3-(3-benzyl-1-imidazoliomethyl)-3-cephem-1-oxido-4-carboxylic acid p-methoxybenzyl ester•iodide (syn-isomer) was obtained 38.9mg of 7-{2-(2-aminothiazole-4-yl)-2-[potassium(2,2-dimethylacetate)oxyimino]acetamide}-3-(3-benzyl-1-imidazoliomethyl)-3-cephem-4-carboxylate (syn-isomer).
NMR δ ppm (DMSO-$d_6$):
1.34(9H,s), 1.43(6H,s), 3.50(2H,s), 5.45(2H,s), 5.68(1H,m), 6.72(1H,s), 7.12(2H,s), 7.42(5H,s), 7.64(1H,s), 8.03(1H,s), 9.65(1H,s), 12.01(1H,m)

Example 34

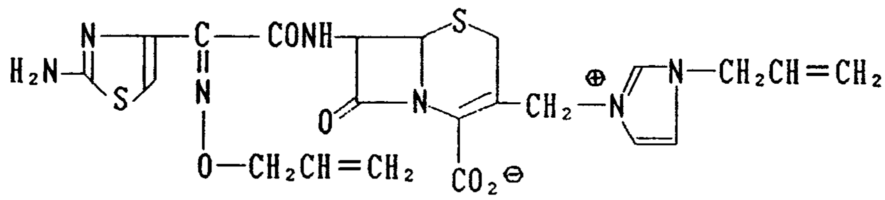

In 20ml of dry ethyl acetate was suspended 0.56g of 7-amino-3-[3-(2-propenyl)-1-imidazoliomethyl]-3-cephem-4-carboxylic acid which was obtained from 7-phenylacetamide-3-[3-(2-propenyl)-1-imidazoliomethyl]-3-cephem-4-carboxylic acid p-methoxybenzyl ester (syn-isomer) by a conventional iminoether method. To the suspension was added 4.8g of bis(trimethylsilyl)acetamide and the mixture was stirred at room temperature (A-solution). Phosphorus oxychloride (0.6g) was added with ice-cooling to 2-propenyloxyimino-2-(2-aminothiazole-4-yl)aceticacid (syn-isomer) (0.53g) and the mixture was stirred for 20 minutes (B-solution).

To A-solution was added dropwise B-solution at -20°C and the mixture was stirred at -10°C to -20°C for 1.5 hours. To the reaction mixture was added 30ml of ice-water with cooling at -20°C to -30°C, and further added 50ml of ethyl acetate. The mixture was stirred and the insolubles were filtered off to obtain an organic layer. To the organic layer was added a saturated aqueous solution of sodium hydrogen carbonate to adjust a pH to 7.5. The separated aqueous layer was washed with methylene chloride. The aqueous layer was adjusted to pH 2 with 10% hydrochloric acid and precipitates were collected by filtration, dried and dissolved into 5% aqueous solution of potassium hydrogen carbonate. The solution was passed through Sephadex LH-20 ($H_2O$) to obtain 18mg of the same compound as in Example 7, ie, 7-[2-(2-aminothiazole-4-yl)-2-(2-propenoxyimino)acetamide]-3-[3-(2-propenyl)-1-imidazoliomethyl]-3-cephem-4-carboxylate (syn-isomer).

Example 35

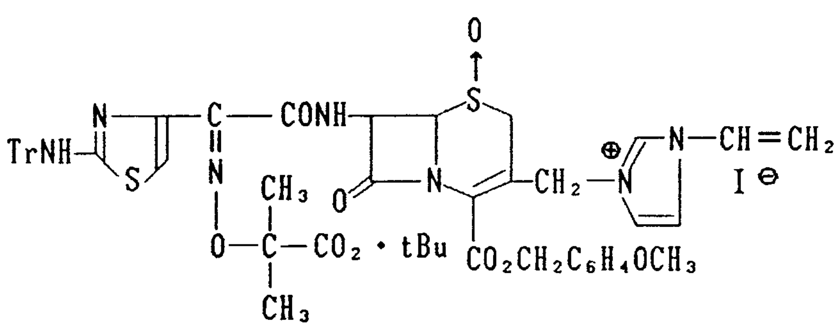

In the same manner as in Example 26, from 500mg of 7-[2-(2-tert-butoxycarbonyl)prop-2-oxyimino-2-(2-tritylaminothiazole-4-yl)acetamide]-3-iodomethyl-3-cephem-1-oxido-4-carboxylic acid p-methoxybenzyl ester (syn-isomer) and 68mg of 1-vinylimidazole was obtained 317.5mg of 7-[2-(2-tert-butoxycarbonyl)prop-2-oxyimino-2-(2-tritylaminothiazole-4-yl)acetamide]-3-(3-vinyl-1-imidazoliomethyl)-3-cephem-1-oxido-4-carboxylic acid p-methoxybenzyl ester. iodide (syn-isomer).

NMR $\delta$ ppm ($CDCl_3$):

1.39(9H,s), 1.55(6H,s), 3.71(3H,s), 4.07(2H,m), 4.97(1H,d,J=5.0Hz), 4.75~5.91(4H,m), 5.21(2H,s), 6.10-(1H,dd,J=5.0,8.0Hz), 6.72(1H,s), 7.05(4H,dd,J=8.0,35.0Hz), 6.61~8.09(5H,m), 7.25(15H,s), 9.68(1H,s)

Example 36

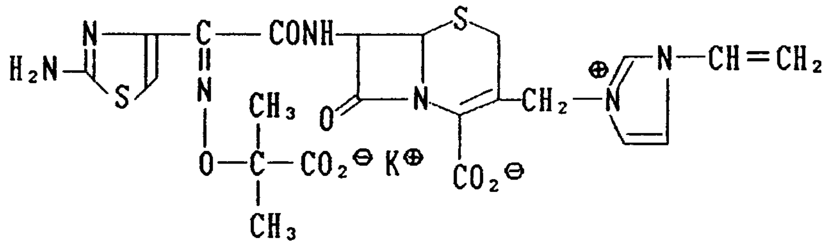

In the same manner as in Examples 27 and 28, from 300mg of 7-[2-(2-tert-butoxycarbonyl)prop-2-oxyimino-2-(2-tritylaminothiazole-4-yl)acetamide]-3-(3-vinyl-1-imidazoliomethyl)-3-cephem-1-oxido-4-carboxylic acid p-methoxybenzyl ester•iodide (syn-isomer) was obtained 25.9mg of 7-{2-(2-aminothiazole-4-yl)-2-[potassium(2,2-dimethylacetate)oxyimino]acetamide}-3-(3-vinyl-1-imidazoliomethyl)-3-cephem-4-carboxylate (syn-isomer).

NMR δ ppm (DMSO-$d_6$):

1.38(3H,s), 1.44(3H,s), 3.10~3.77(2H,m), 4.65~5.50(4H,m), 4.98(1H,d,J = 5.0Hz), 5.64(1H,dd,J = 5.0,8.0Hz), 6.75(1H,s), 6.90~7.60(1H,m), 7.12(1H,s), 7.98(1H,s), 8.11(1H,s), 9.70(1H,s), 12.06(1H,d,J = 8.0Hz)

Example 37

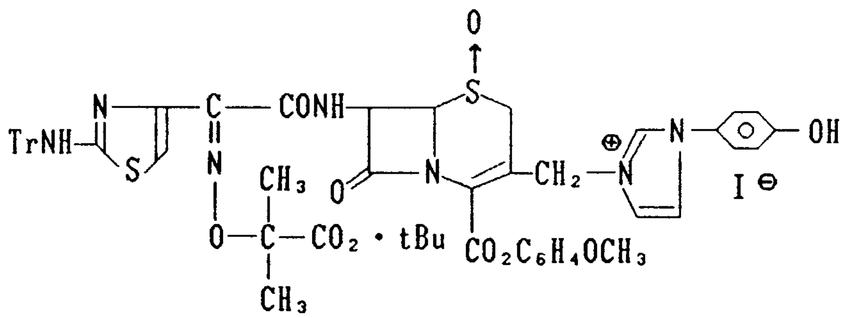

In the same manner as in Example 26, from 350mg of 7-[2-(2-tert-butoxycarbonyl)prop-2-oxyimino-2-(2-tritylaminothiazole-4-yl)acetamide]-3-iodomethyl-3-cephem-1-oxido-4-carboxylic acid p-methoxybenzyl ester (syn-isomer) and 65mg of 1-(4-hydroxyphenyl)imidazole was obtained 160.9mg of 7-[2-(2-tert-butoxycarbonyl)prop-2-oxyimino-2-(2-trityl aminothiazole-4-yl)acetamide]-3-[3-(4-hydroxyphenyl)-1-imidazoliomethyl]-3-cephem-1-oxido-4-carboxylic acid p-methoxybenzyl ester•iodide (syn-isomer).

NMR δ ppm ($CDCl_3$):

1.37(9H,s), 1.54(6H,s), 3.76(3Hs), 4.13(2H,m), 4.99(1H,d,J = 5.0Hz), 5.24(2H,s), 5.05~5.85(2H,m), 6.14-(1H,dd,J = 5.0,8.0Hz), 6.63(1H,s), 7.07(4H,dd,J = 8.0, 37.5Hz), 6.65~8.10(9H,m), 7.23(15H,s), 9.71(1H,s)

Example 38

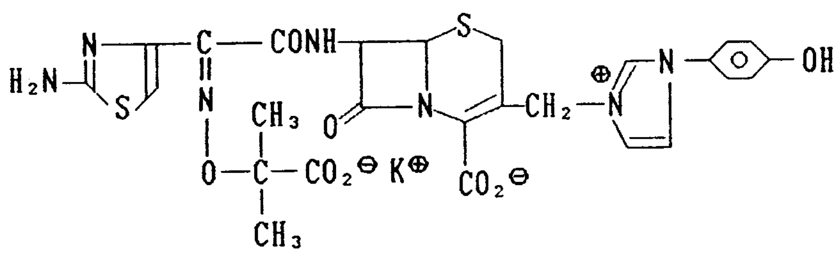

In the same manner as in Examples 27 and 28, from 160mg of 7-[2-(2-tert-butoxycarbonyl)prop-2-

oxyimino-2-(2-tritylaminothiazole-4-yl)acetamide]-3-[3-(4-hydroxyphenyl)-1-imidazoliomethyl]-3-cephem-1-oxido-4-carboxylic acid p-methoxybenzyl ester•iodide (syn-isomer) was obtained 9.0mg of 7-{2-(2-aminothiazole-4-yl)-2-[potassium(2,2-dimethylacetate)oxyimino]acetamide}-3-[3-(4-hydroxyphenyl)-1-imidazoliomethyl]-3-cephem-4-carboxylate (syn-isomer).
NMR δ ppm (DMSO-$d_6$):
1.39(3H,s), 1.41(3H,s), 3.20~3.72(2H,m), 4.95(1H,d,J=5.0Hz), 5.05~5.40(2H,m), 5.47~5.84(1H,m), 6.72-(1H,s), 6.23~8.25(9H,m), 9.47(1H,s), 12.03(1H,d,J=8.0Hz)

Example 39

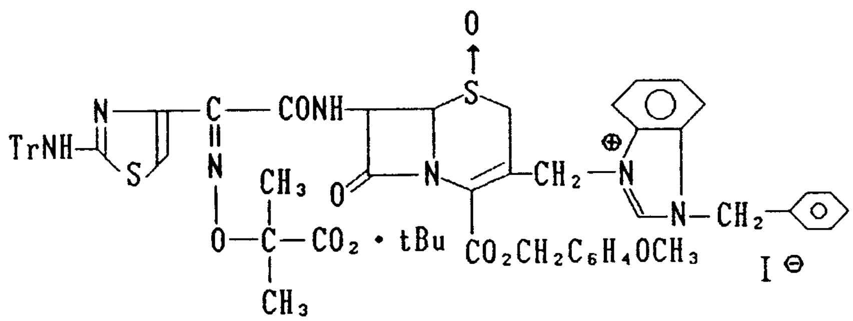

In the same manner as in Example 26, from 500mg of 7-[2-(2-tert-butoxycarbonyl)prop-2-oxyimino-2-(2-tritylaminothiazole-4-yl)acetamide]-3-iodomethyl-3-cephem-1-oxido-4-carboxylic acid p-methoxybenzyl ester (syn-isomer) and 121mg of 1-benzylbenzimidazole was obtained 288.5mg of 7-[2-(2-tert-butoxycarbonyl)-prop-2-oxyimino-2-(2-tritylaminothiazole-4-yl)acetamide]-3-(benz-3-N-benzyl-1-imidazoliomethyl)-3-cephem-1-oxido-4-carboxylic acid p-methoxybenzyl ester•iodide (syn-isomer).
NMR δ ppm ($CDCl_3$):
1.39(9H,s), 1.54(6H,s), 3.74(3H,s), 4.16(2H,m), 5.13(1H,d,J=5.0Hz), 5.23(2H,s), 6.10(2H,s), 5.75(2H,m), 6.11-(1H,dd,J=5.0,8.0Hz), 7.02(4H,dd,J=8.0,42.5Hz), 7.25(15H,s), 7.37(5H,s), 6.70~8.10(7H,m)

Example 40

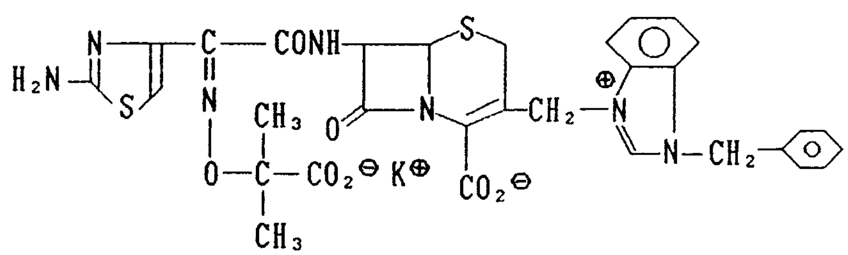

In the same manner as in Examples 27 and 28, from 280mg of 7-[2-(2-tert-butoxycarbonyl)prop-2-oxyimino-2-(2-tritylaminothiazole-4-yl)acetamide]-3-(benz-3-N-benzyl-1-imidazoliomethyl)-3-cephem-1-oxido-4-carboxylic acid p-methoxybenzyl ester•iodide (syn-isomer) was obtained 30.1mg of 7-{2-(2-aminothiazole-4-yl)-2-[potassium(2,2-dimethylacetate)oxyimino]acetamide}-3-(benz-3-N-benzyl-1-imidazoliomethyl)-3-cephem-4-carboxylate (syn-isomer).
NMR δ ppm (DMSO-$d_6$):
1.35(3H,s), 1.40(3H,s), 3.08~3.76(2H,m), 4.93(1H,d,J=5.0Hz), 5.45(2H,s), 5.77(2H,s), 5.46~5.82(1H,m), 6.66-(1H,s), 6.85~8.50(9H,m), 7.07(2H,s), 10.13(1H,s), 11.84(1H,d,J=8.0Hz)

Example 41

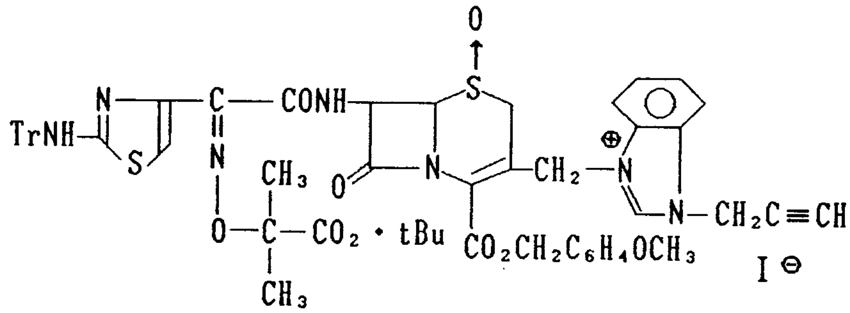

In the same manner as in Example 26, from 350mg of 7-[2-(2-tert-butoxycarbonyl)prop-2-oxyimino-2-(2-tritylaminothiazole-4-yl)acetamide]-3-iodomethyl-3-cephem-1-oxido-4-carboxylic acid p-methoxybenzyl ester (syn-isomer) and 64mg of 1-(2-propynyl)benzimidazole was obtained 209.1mg of 7-[2-(2-tert-butoxycarbonyl)prop-2-oxyimino-2-(2-tritylaminothiazole-4-yl)acetamide]-3-[benz-3-N-(2-propynyl)-1-imidazoliomethyl]-3-cephem-1-oxido-4-carboxylic acid p-methoxybenzyl ester•iodide (syn-isomer).

NMR δ ppm ($CDCl_3$):

1.38(9H,s), 1.52(6H,s), 2.47(1H,t,J=2.8Hz), 3.72(3H,s), 4.07(2H,m), 4.88(2H,d,J=2.8Hz), 5.16-(1H,d,J=5.0Hz), 5.25(2H,s), 6.03(2H,m), 6.23(1H,dd,J=5.0,8.0Hz), 6.59(1H,s), 7.01(4H,dd,J=8.0,42.5Hz), 7.26(15H,s), 6.62~8.10(6H,m), 10.31(1H,s)

Example 42

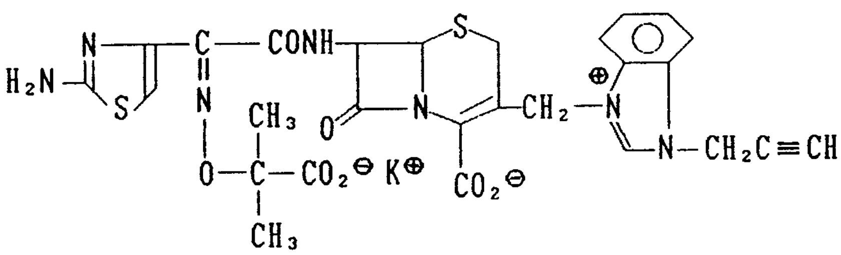

In the same manner as in Examples 27 and 28, from 217.6mg of 7-[2-(2-tert-butoxycarbonyl)prop-2-oxyimino-2-(2-tritylaminothiazole-4-yl)acetamide]-3-[benz-3-N-(2-propynyl)-1-imidazoliomethyl]-3-cephem-1-oxido-4-carboxylic acid p-methoxybenzyl ester•iodide (syn-isomer) was obtained 36.5mg of 7-{2-(2-aminothiazole-4-yl)-2-[potassium(2,2-dimethylacetate)oxyimino]acetamide}-3-[benz-3-N-(2-propynyl)-1-imidazoliomethyl]-3-cephem-4-carboxylate (syn-isomer).

NMR δ ppm (DMSO-$d_6$):

1.38(3H,s), 1.42(3H,s), 3.35~3.86(2H,m), 4.96(1H,d,J=5.0Hz), 5.05~5.82(5H,m), 6.67(1H,s), 7.10(2H,s), 6.96~8.50(4H,m), 10.06(1H,s), 11.17(1H,d,J=8.0Hz)

Example 43

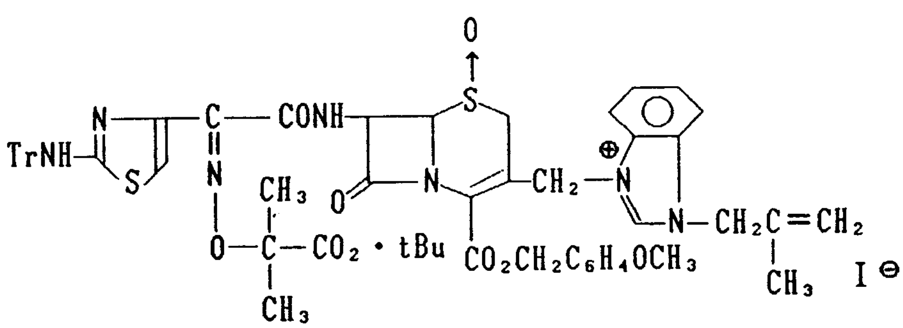

In the same manner as in Example 26, from 500mg of 7-[2-(2-tert-butoxycarbonyl)prop-2-oxyimino-2-(2-tritylaminothiazole-4-yl)acetamide]-3-iodomethyl-3-cephem-1-oxido-4-carboxylic acid p-methoxybenzyl ester (syn-isomer) and 100mg of 1-(2-methyl-2-propenyl)benzimidazole was obtained 268.9mg of 7-[2-(2-tert-butoxycarbonyl)prop-2-oxyimino-2-(2-tritylaminothiazole-4-yl)acetamide]-3-[benz-3-N-(2-methyl-2-propenyl)-1-imidazoliomethyl]-3-cephem-1-oxido-4-carboxylic acid p-methoxybenzyl ester•iodide (syn-isomer).

NMR δ ppm ($CDCl_3$):

1.40(9H,s), 1.54(6H,s), 1.75(3H,s), 2.76(3H,s), 4.17(2H,m), 4.66(2H,s), 4.85~5.40(3H,m), 5.21(2H,s), 5.75-(2H,m), 6.20(1H,dd,J = 5.0,8.0Hz), 6.59(1H,s), 7.02(4H,dd,J = 8.0,42.5Hz), 7.25(15H,s), 6.70~8.15(7H,m)

Example 44

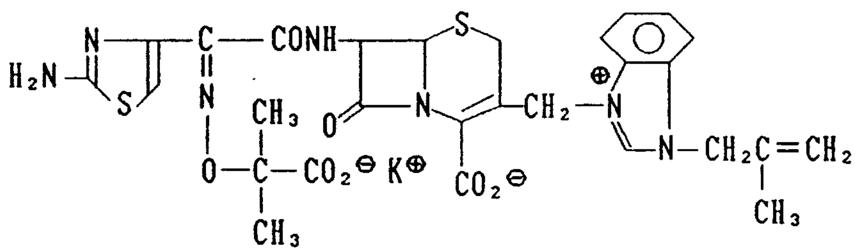

In the same manner as in Examples 27 and 28, from 265mg of 7-[2-(2-tert-butoxycarbonyl)prop-2-oxyimino-2-(2-tritylaminothiazole-4-yl)acetamide]-3-[benz-3-N-(2-methyl-2-propenyl-1-imidazoliomethyl]-3-cephem-1-oxido-4-carboxylic acid p-methoxybenzyl ester•iodide (syn-isomer) was obtained 30.8mg of 7-{2-(2-aminothiazole-4-yl)-2-[potassium(2,2-dimethylacetate)oxyimino]acetamide}-3-[benz-3-N-(2-methyl-2-propenyl)-1-imidazoliomethyl]-3-cephem-4-carboxylate (syn-isomer).

NMR δ ppm (DMSO-$d_6$):

1.37(3H,s), 1.42(3H,s), 2.24(3H,s), 3.32~3.75(2H,m), 4.55~5.25(2H,m), 4.95(1H,d,J = 5.0Hz), 5.15(2H,s), 5.42-(2H,s), 5.62(1H,dd,J = 5.0,8.0Hz), 6.67(1H,s), 7.07(2H,s), 6.81~8.46(4H,m), 9.99(1H,s), 11.58(1H,d,J = 8.0Hz)

Example 45

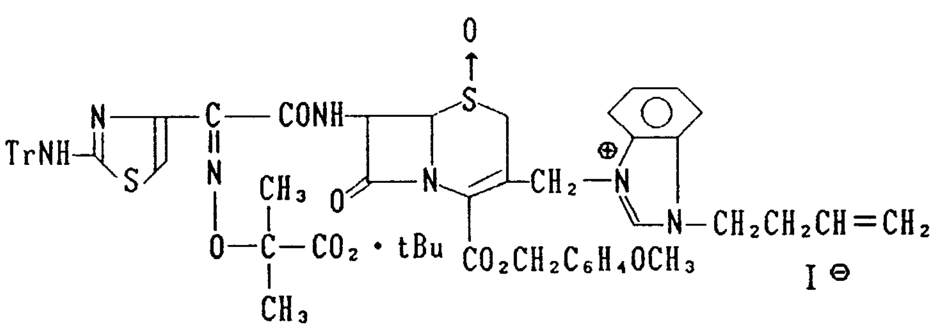

In the same manner as in Example 26, from 350mg of 7-[2-(2-tert-butoxycarbonyl)prop-2-oxyimino-2-(2-tritylaminothiazole-4-yl)acetamide]-3-iodomethyl-3-cephem-1-oxido-4-carboxylic acid p-methoxybenzyl ester (syn-isomer) and 70mg of 1-(3-butenyl)benzimidazole was obtained 214mg of 7-[2-(2-tert-butoxycarbonyl)-prop-2-oxyimino-2-(2-tritylaminothiazole-4-yl)acetamide]-3-[benz-3-N-(3-butenyl)-1-imidazoliomethyl]-3-cephem-1-oxido-4-carboxylic acid p-methoxybenzyl ester•iodide (syn-isomer).

NMR δ ppm ($CDCl_3$):

1.38(9H,s), 1.52(6H,s), 2.72(2H,q,J = 6.0Hz), 3.80(3H,s), 4.14(2H,m), 4.48(2H,t,J = 6.0Hz), 5.20(2H,s), 4.80~5.26(3H,m), 5.67(2H,m), 5.70~6.20(1H,m), 6.17(1H,dd,J = 5.0,8.0Hz), 6.58(1H,s), 7.02-(4H,dd,J = 80,42.5Hz), 7.24(15H,s), 6.75~8.08(6H,m), 10.6(1H,s)

Example 46

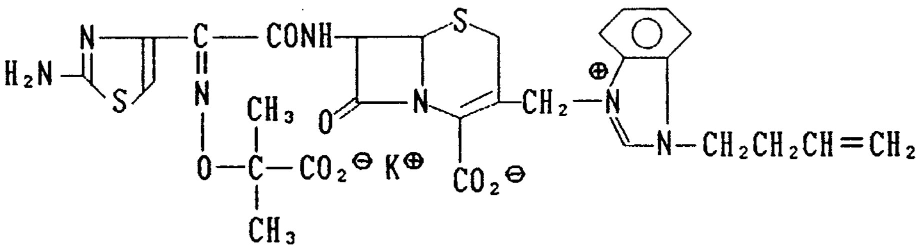

In the same manner as in Examples 27 and 28, from 210mg of 7-[2-(2-tert-butoxycarbonyl)prop-2-oxyimino-2-(2-tritylaminothiazole-4-yl)acetamide]-3-[benz-3-N-(3-butenyl)-1-imidazoliomethyl]-3-cephem-1-oxido-4-carboxylic acid p-methoxybenzyl ester•iodide (syn-isomer) was obtained 25.3mg of 7-{2-(2-aminothiazole-4-yl)-2-[potassium(2,2-dimethylacetate)oxyimino]acetamide}-3-[benz-3-N-(3-butenyl)-1-imidazoliomethyl]-3-cephem-4-carboxylate (syn-isomer).

NMR δ ppm (DMSO-$d_6$):

1.36(3H,s), 1.41(3H,s), 2.66(2H,q,J = 6.0Hz), 3.30~3.75(2H,m), 4.58(2H,t,J = 6.0Hz), 4.92(1H,d,J = 5.0Hz), 5.04(2H,s), 5.25~6.22(4H,m), 6.67(1H,s), 7.07(2H,s), 7.35~8.47(4H,m), 10.3(1H,s), 11.91(1H,d,J = 8.0Hz)

Example 47

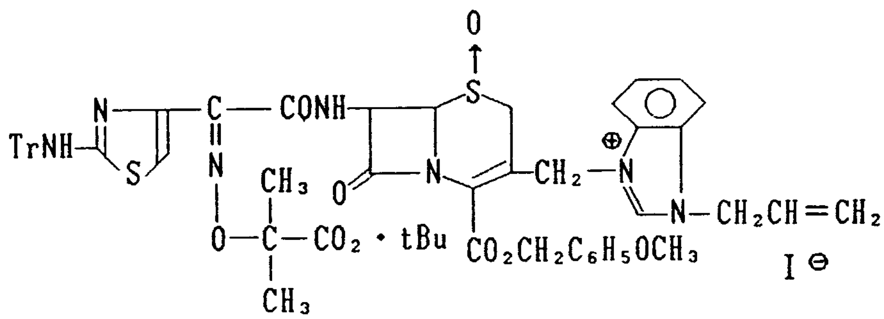

In the same manner as in Example 26, from 350mg of 7-[2-(2-tert-butoxycarbonyl)prop-2-oxyimino-2-(2-tritylaminothiazole-4-yl)acetamide]-3-iodomethyl-3-cephem-1-oxido-4-carboxylic acid p-methoxybenzyl ester (syn-isomer) and 65mg of 1-(2-propenyl)benzimidazole was obtained 224.3mg of 7-[2-(2-tert-butoxycarbonyl)prop-2-oxyimino-2-(2-tritylaminothiazole-4-yl)acetamide]-3-[benz-3-N-(2-propenyl)-1-imidazoliomethyl]-3-cephem-1-oxido-4-carboxylic acid p-methoxybenzyl ester•iodide (syn-isomer).

NMR δ ppm ($CDCl_3$):

1.40(9H,s), 1.54(6H,s), 3.66(3H,s), 4.11(2H,m), 4.76(2H,d,J = 6.3Hz), 5.02(1H,d,J = 5.0Hz), 5.23(2H,s), 5.05~6.14(2H,m), 6.37~6.70(4H,m), 6.61(1H,s), 7.04(4H,dd,J = 8.0,42.5Hz), 7.26(15H,s), 6.65~8.15(7H,m)

Example 48

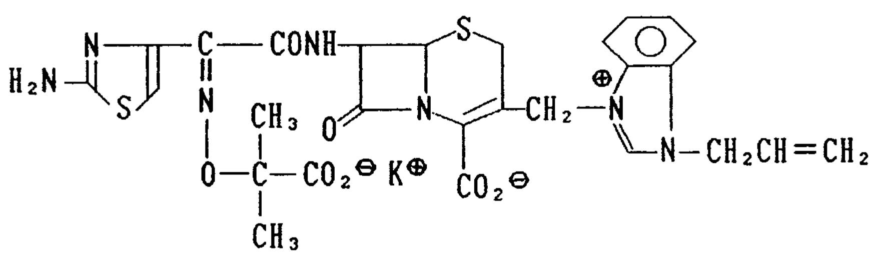

In the same manner as in Example 27 and 28, from 200mg of 7-[2-(2-tert-butoxycarbonyl)prop-2-

oxyimino-2-(2-tritylaminothiazole-4-yl)acetamide]-3-[benz-3-N-(2-propenyl)-1-imidazoliomethyl]-3-cephem-1-oxido-4-carboxylic acid p-methoxybenzyl ester•iodide (syn-isomer) was obtained 11.5mg of 7-{2-(2-aminothiazole-4-yl)-2-[potassium(2,2-dimethylacetate)oxyimino]acetamide}-3-[benz-3-N-(2-propenyl)-1-imidazoliomethyl]-3-cephem-4-carboxylate (syn-isomer).

NMR δ ppm (DMSO-$d_6$):

1.37(3H,s), 1.39(3H,s), 3.12~3.77(2H,m), 4.87(2H,d,J = 6.0Hz), 4.93(1H,d,J = 5.0Hz), 5.02~5.74(3H,m), 5.40-(2H,s), 5.74~6.34(1H,m), 6.65(1H,s), 6.41~8.46(4H,m), 7.10(2H,s), 9.91(1H,s), 11.90(1H,d,J = 8.0Hz)

Example 49

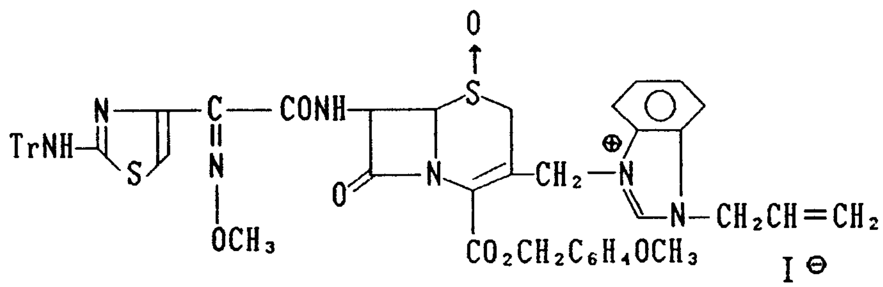

In the same manner as in Example 4, from 450mg of 7-[2-methoxyimino-2-(2-tritylaminothiazole-4-yl)-acetamide]-3-iodomethyl-3-cephem-1-oxido-4-carboxylic acid p-methoxybenzyl ester (syn-isomer) and 87mg of 1-(2-propenyl)benzimidazole was obtained 130mg of 7-[2-methoxyimino-2-(2-tritylaminothiazole-4-yl)-acetamide]-3-[benz-3-N-(2-propenyl)-1-imidazoliomethyl]-3-cephem-1-oxido-4-carboxylic acid p-methoxybenzyl ester•iodide (syn-isomer).

NMR δ ppm (DMSO-$d_6$):

3.75(3H,s), 3.80(3H,s), 4.1~4.3(2H,m), 4.45(2H,s), 4.90~5.10(2H,m), 5.22(4H,s), 5.45(2H,s), 5.5~5.65(1H,m), 6.75(1H,s), 7.25(15H,s), 9.20(1H,s)

Example 50

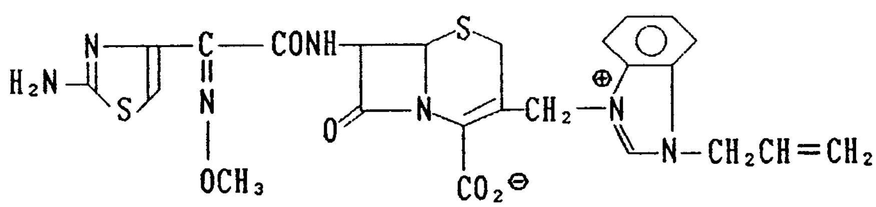

In the same manner as in Examples 5, 6 and 7, from 120mg of 7-[2-methoxyimino-2-(2-tritylaminothiazole-4-yl)acetamide]-3-[benz-3-N-(2-propenyl)-1-imidazoliomethyl]-3-cephem-1-oxido-4-carboxylic acid p-methoxybenzyl ester•iodide (syn-isomer) was obtained 17mg of 7-[2-(2-aminothiazole-4-yl)-2-methoxyiminoacetamide]-3-[benz-3-N-(2-propenyl)-1-imidazoliomethyl]-3-cephem-4-carboxylate (syn-isomer).

NMR δ ppm (DMSO-$d_6$):

3.10~3.70(2H,m), 3.80(3H,s), 4.1~4.3(2H,m), 4.40~4.50(2H,m), 4.98(2H,d,J = 8.0Hz), 5.22~5.45(4H,m), 6.75-(1H,s), 12.5(1H,d,J = 8.0Hz)

Example 51

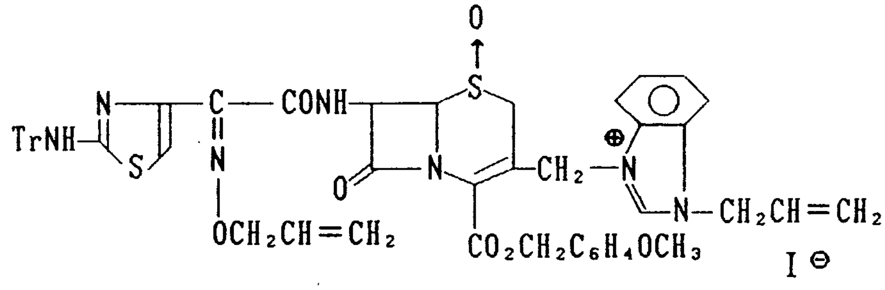

In the same manner as in Example 4, from 460mg of 7-[2-(2-propenyloxyimino)-2-(2-tritylaminothiazole-4-yl)acetamide]-3-iodomethyl-3-cephem-1-oxido-4-carboxylic acid p-methoxybenzyl ester (syn-isomer) and 87mg of 1-(2-propenyl)benzimidazole was obtained 140mg of 7-[2-(2-propenyloxyimino)-2-(2-tritylaminothiazole-4-yl)acetamide]-3-[benz-3-N-(2-propenyl)-1-imidazoliomethyl]-3-cephem-1-oxido-4-carboxylic acid p-methoxybenzyl ester•iodide (syn-isomer).

NMR δ ppm (DMSO-$d_6$):

3.75(3H,s), 4.2~4.3(4H,m), 4.45(2H,s), 5.05~5.15(4H,m), 5.22~5.45(6H,m), 5.5~5.6(2H,m), 6.75(1H,s), 7.08~7.15(4H,m), 7.25(15H,s), 9.20(1H,s)

Example 52

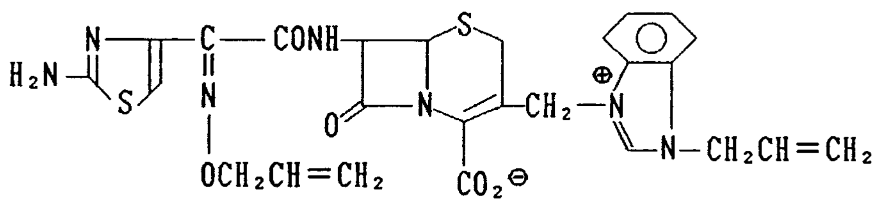

In the same manner as in Examples 5, 6 and 7, from 130mg of 7-[2-(2-propenyloxyimino)-2-(2-tritylaminothiazole-4-yl)acetamide]-3-[benz-3-N-(2-propenyl)-1-imidazoliomethyl]-3-cephem-1-oxido-4-carboxylic acid p-methoxybenzyl ester•iodide (syn-isomer) was obtained 26mg of 7-[2-(2-aminothiazole-4-yl)-2-(2-propenyloxyimino)acetamide]-3-[benz-3-N-(2-propenyl)-1-imidazoliomethyl]-3-cephem-4-carboxylate (syn-isomer).

NMR δ ppm (DMSO-$d_6$):

3.60~3.65(2H,m), 4.2~4.3(4H,m), 4.45(2H,s), 5.05~5.15(4H,m), 5.22~5.45(4H,m), 5.5~5.6(2H,m), 6.75(1H,s), 12.5(1H,d,J = 8.0Hz)

Example 53

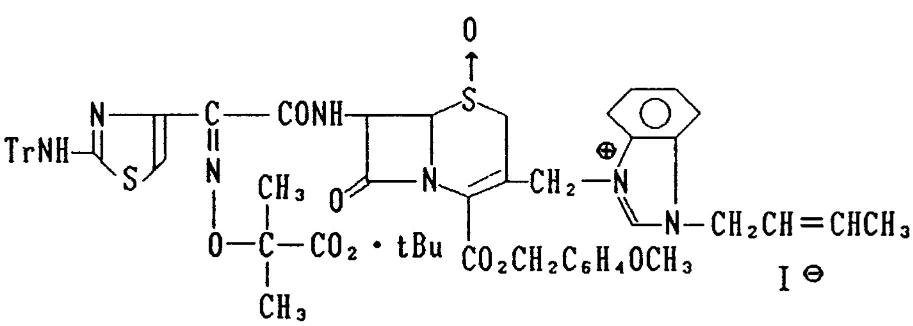

In the same manner as in Example 26, from 502mg of 7-[2-(2-tert-butoxycarbonyl)prop-2-oxyimino-2-(2-tritylaminothiazole-4-yl)acetamide]-3-iodomethyl-3-cephem-1-oxido-4-carboxylic acid p-methoxybenzyl ester (syn-isomer) and 103mg of 1-(2-butenyl)benzimidazole was obtained 410mg of 7-[2-(2-tert-butoxycarbonyl)-prop-2-oxyimino-2-(2-tritylaminothiazole-4-yl)acetamide]-3-[benz-3-N-(2-butenyl)-1-imidazoliomethyl]-3-cephem-1-oxido-4-carboxylic acid p-methoxybenzyl ester•iodide (syn-isomer).
NMR δ ppm (DMSO-$d_6$):
1.34(9H,s), 1.44(3H,s), 1.7(3H,s), 4.7~4.85(3H,m), 5.30(2H,s), 5.5~5.9(3H,m), 6.60(1H,s), 7.25(15H,s), 9.20-(1H,s)

Example 54

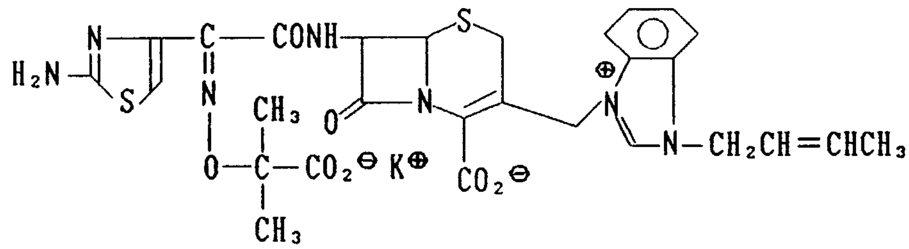

In the same manner as in Examples 27 and 28, from 280mg of 7-[2-(2-tert-butoxycarbonyl)prop-2-oxyimino-2-(2-tritylaminothiazole-4-yl)acetamide]-3-[benz-3-N-(2-butenyl)-1-imidazoliomethyl]-3-cephem-1-oxido-4-carboxylic acid p-methoxybenzyl ester•iodide (syn-isomer) was obtained 21.5mg of 7-{2-(2-aminothiazole-4-yl)-2-[potassium(2,2-dimethylacetate) oxyimino]acetamide}-3-[benz-3-N-(2-butenyl)-1-imidazoliomethyl]-3-cephem-4-carboxylate (syn-isomer).
NMR δ ppm (DMSO-$d_6$):
1.38(3H,s), 1.41(3H,s), 1.7(3H,s), 4.7~4.85(3H,m), 5.30(2H,s), 5.5~5.9(3H,m), 6.60(1H,s), 9.80(1H,s), 12.50-(1H,d,J=8.0Hz)

Example 55

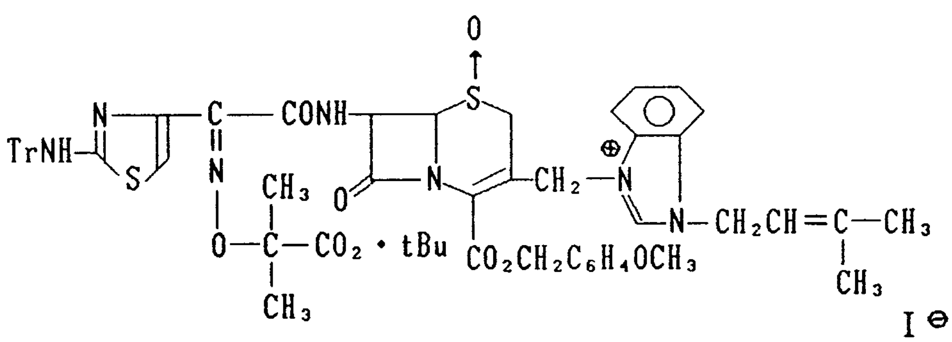

In the same manner as in Example 26, from 502mg of 7-[2-(2-tert-butoxycarbonyl)prop-2-oxyimino-2-(2-tritylaminothiazole-4-yl)acetamide]-3-iodomethyl-3-cephem-1-oxido-4-carboxylic acid p-methoxybenzyl ester (syn-isomer) and 111mg of 1-(3-methyl-2-butenyl)benzimidazole was obtained 380mg of 7-[2-(2-tert-butoxycarbonyl)prop-2-oxyimino-2-(2-tritylaminothiazole-4-yl)acetamide]-3-[benz-3-N-(3-methyl-2-butenyl)-1-imidazoliomethyl]-3-cephem-1-oxido-4-carboxylic acid p-methoxybenzyl ester•iodide (syn-isomer).
NMR δ ppm (DMSO-$d_6$):
1.34(9H,s), 1.39(6H,s), 1.80(6H,s), 4.7~4.85(3H,m), 5.30(2H,s), 5.3~5.6(2H,m), 6.60(1H,s), 7.25(15H,s), 9.20-(1H,s)

Example 56

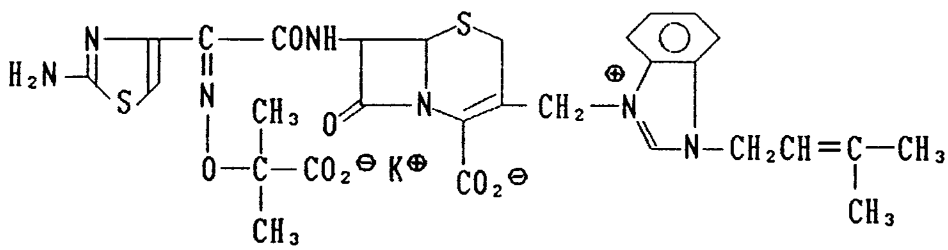

In the same manner as in Examples 27 and 28, from 260mg of 7-[2-(2-tert-butoxycarbonyl)prop-2-oxyimino-2-(2-tritylaminothiazole-4-yl)acetamide]-3-[benz-3-N-(3-methyl-2-butenyl)-1-imidazoliomethyl]-3-cephem-1-oxido-4-carboxylic acid p-methoxybenzyl ester•iodide (syn-isomer) was obtained 12.6mg of 7-{2-(2-aminothiazole-4-yl)-2-[potassium(2,2-dimethylacetate)oxyimino]acetamide}-3-[benz-3-N-(3-methyl-2-butenyl)-1-imidazoliomethyl]-3-cephem-4-carboxylate (syn-isomer).

NMR δ ppm (DMSO-$d_6$):

1.38(6H,s), 1.80(6H,s), 4.7~4.85(3H,m), 5.30(2H,s), 5.3~5.6(2H,m), 6.60(1H,s), 9.80(1H,s), 12.50-(1H,d,J = 8.0Hz)

Example 57

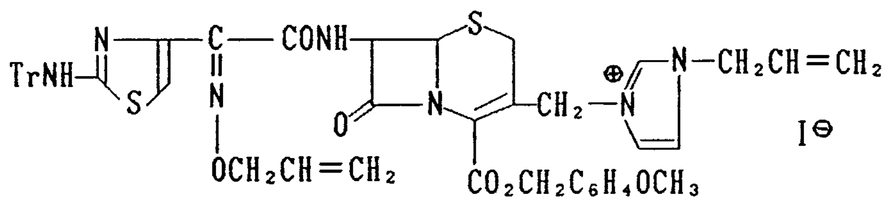

Into a solvent mixture of chloroform (600ml) and acetonitrile (1000ml) was dissolved 214.4g of 7-[2-(2-propenyloxyimino)-2-(2-tritylaminothiazole-4-yl)acetamide]-3-iodomethyl-3-cephem-4-carboxylic acid p-methoxybenzyl ester (syn-isomer) obtained in Example 2. To the solution was added with ice-cooling 41.1g of 1-(2-propenyl)imidazole. After the addition, the mixture was heated to room temperature and was further stirred for 4 hours. Solvents were removed at a reduced pressure. The residue was purified by a silicagel column (chloroform/methanol = 3/1, volume ratio) to collect eluate containing a desired compound. By removing a solvent was obtained 268g of 7-[2-(2-propenyloxyimino)-2-(2-tritylaminothiazole-4-yl)acetamide)-3-[3-(2-propenyl)-1-imidazoliomethyl)-3-cephem-4-carboxylic acid p-methoxybenzyl ester•iodide (syn-isomer).

NMR δ ppm (DMSO-$d_6$):

3.55(2H,s), 3.77(3H,s), 4.58(2H,d,J = 5.0Hz), 4.87(2H,d,J = 5.0Hz), 4.98~5.60(9H,m), 5.62~6.35(3H,m), 6.76-(1H,s), 7.12(4H,dd,J = 8.0Hz,32.5Hz), 7.32(15H,s), 7.68(1H,s), 7.75(1H,s), 9.28(1H,s), 9.27(1H,s), 9.58-(1H,d,J = 8.0Hz)

In order to demonstrate use of Compound (I) of the invention, typical compounds of the formula (I) were tested for in vitro anti-bacterial activity. The results were shown below.

Test: in vitro anti-bacterial activity

Compounds tested:

- A: Compound of Example 7
- B: Compound of Example 13
- C: Compound of Example 15
- D: Compound of Example 17
- E: Compound of Example 21
- F: Compound of Example 23

G: Compound of Example 25
H: Compound of Example 28
I: Compound of Example 30
K: Compound of Example 32
M: Compound of Example 36
O: Compound of Example 38
P: Compound of Example 40
Q: Compound of Example 42
R: Compound of Example 44
S: Compound of Example 46
U: Compound of Example 48
V: Compound of Example 50
W: Compound of Example 52
X: Compound of Example 54
Y: Compound of Example 56

Test method:

The agent for preventing and treating bacterial infections was tested for in vitro anti-bacterial activity by the following two-fold agar plate dilution method. Each test strain was incubated for 20 hours in a bouillon for measuring sensitivity to obtain a test culture (containing about $10^8$ live cells/ml). Portions of sensitivity measuring agar medium containing varying concentrations of the antibacterial agent were each innoculated with a 0.005ml portion of the culture, followed by incubation at 37°C for 20 hours. The minimum growth inhibitory concentration (MIC) in µg/ml was then determined. Table 1 shows the result.

MIC = minimum inhibitory concentration

Table 1

MIC (μg/ml)

| Compound | A | B | C | D | E | F |
|---|---|---|---|---|---|---|
| Staphylococcus aureus 209P | 0.78 | 0.20 | 0.39 | 0.78 | 0.78 | 6.25 |
| Escherichia coli NIHJ-JC2 | 0.78 | 0.39 | 0.39 | 0.78 | 0.20 | 0.1 |
| Klebsiella pneumoniae | 1.56 | 0.39 | 0.78 | 0.78 | 0.20 | 0.1 |
| Proteus vulgaris | 1.56 | 0.78 | 0.78 | 1.56 | 0.39 | 0.39 |
| Proteus mirabilis | 3.13 | 0.78 | 0.78 | 1.56 | 0.78 | 0.39 |
| Serratia marcescens | 0.78 | 1.56 | 3.12 | 3.13 | 1.56 | 0.78 |
| Escherichia coli CSH (RE45) | 0.39 | 0.10 | 0.20 | 0.78 | 0.05 | 0.2 |
| Pseudomonas aeruginosa | 6.25 | 6.25 | 12.5 | 12.5 | 12.5 | 25 |
| Pseudomonas cepacia | 6.25 | 1.56 | 6.25 | 12.5 | >50 | 50 |

Table 1 (continued)

MIC (μg/ml)

| Compound | G | H | I | K |
|---|---|---|---|---|
| Staphylococcus aureus 209P | 0.78 | 5.0 | 6.25 | 12.5 |
| Escherichia coli NIHJ-JC2 | 0.05 | 0.20 | 0.39 | 0.2 |
| Klebsiella pneumoniae | 0.1 | 0.78 | 1.56 | 0.78 |
| Proteus vulgaris | 0.2 | 0.10 | 0.2 | 0.39 |
| Proteus mirabilis | 0.2 | 0.20 | 0.2 | 0.39 |
| Serratia marcescens | 1.56 | 0.78 | 1.56 | 0.78 |
| Escherichia coli CSH (RE45) | 0.2 | 0.10 | 0.2 | 0.39 |
| Pseudomonas aeruginosa | 6.25 | 3.12 | 12.5 | 3.13 |
| Pseudomonas cepacia | 25 | 6.25 | 25 | 0.39 |

Table 1 (continued)

MIC (μg/ml)

| Compound | M | O | P | Q | R |
|---|---|---|---|---|---|
| Staphylococcus aureus 209P | 6.25 | 6.25 | 3.13 | 3.13 | 6.25 |
| Escherichia coli NIHJ-JC2 | 0.2 | 0.2 | 3.13 | 0.2 | 0.78 |
| Klebsiella pneumoniae | 0.39 | 0.78 | 6.25 | 0.78 | 3.13 |
| Proteus vulgaris | 0.1 | 0.1 | 1.56 | 0.1 | 0.78 |
| Proteus mirabilis | 0.1 | 0.2 | 3.13 | 0.39 | 3.13 |
| Serratia marcescens | 0.78 | 0.78 | 6.25 | 0.78 | 1.56 |
| Escherichia coli CSH (RE45) | 0.1 | 0.1 | 1.56 | 0.2 | 1.56 |
| Pseudomonas aeruginosa | 3.13 | 6.25 | 6.25 | 3.13 | 12.5 |
| Pseudomonas cepacia | 12.5 | 25 | 50 | 12.5 | 25 |

Table 1 (continued)

MIC (μg/ml)

| Compound | S | U | V | W | X | Y |
|---|---|---|---|---|---|---|
| Staphylococcus aureus 209P | 0.78 | 6.25 | 3.13 | 1.56 | 6.25 | 6.25 |
| Escherichia coli NIHJ-JC2 | 0.78 | 0.39 | 0.78 | 3.13 | 1.56 | 6.25 |
| Klebsiella pneumoniae | 3.13 | 0.78 | 1.56 | 6.25 | 3.13 | 12.5 |
| Proteus vulgaris | 0.78 | 0.39 | 3.13 | 3.13 | 1.56 | 3.13 |
| Proteus mirabilis | 3.13 | 1.56 | 6.25 | 6.25 | 1.56 | 6.25 |
| Serratia marcescens | 3.13 | 1.56 | 12.5 | 25 | 1.56 | 12.5 |
| Escherichia coli CSH(RE45) | 0.39 | 0.2 | 3.13 | 1.56 | 0.78 | 1.56 |
| Pseudomonas aeruginosa | 6.25 | 6.25 | >100 | 50 | 12.5 | 25 |
| Pseudomonas cepacia | 25 | 25 | >100 | >100 | 50 | >100 |

## Claims

**Claims for the following Contracting States : BE, CH, DE, FR, GB, IT, LI, NL, SE**

**1.** A cephalosporin compound substituted by an imidazolium ring in 3-position of cephem having the formula (I) or (I') and the pharmaceutically acceptable salts thereof:

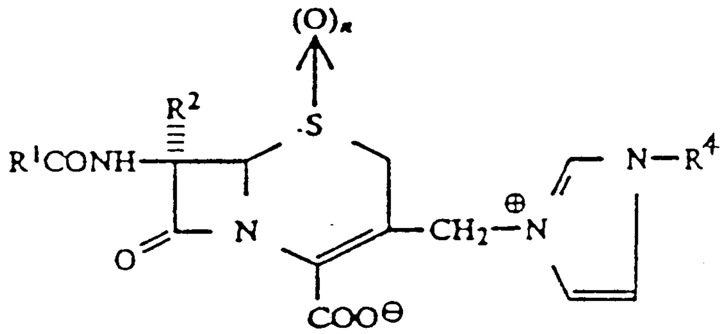

(I)

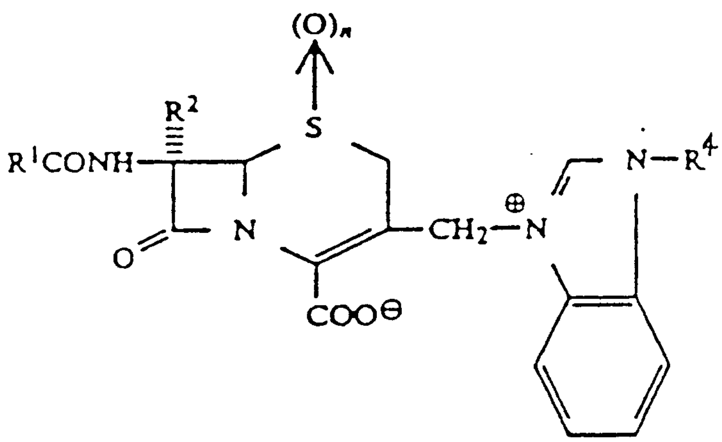

(I')

wherein

$R^1$ is

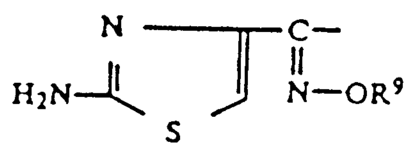

;

$R^2$ is hydrogen or methoxy;

$R^4$ is $C_2$-$C_{12}$ alkenyl, $C_2$-$C_{12}$ alkynyl, benzyl or phenyl;

$R^9$ is $C_1$-$C_6$ alkyl, $C_2$-$C_6$ alkenyl, $C_2$-$C_6$ alkynyl or a group of formula

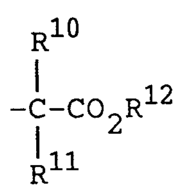

wherein $R^{10}$ and $R^{11}$ are the same or different and represent hydrogen, methyl or ethyl and $R^{12}$ is hydrogen, an alkali metal, alkaline earth metal, organic amine base or protective group for carboxyl; and

n is 0 or 1; The following compounds are also claimed

7-[2-(2-Aminothiazole-4-yl)-2-(2-propenyloxyimino)acetamide]-3-(3-acetophenyl-1-imidazoliomethyl)-3-cephem-4-carboxylate;

7-[2-(2-Aminothiazole-4-yl)-2-(2-propenyloxyimino)acetamide]-3-(3-(4-hydroxyphenyl)-1-imidazoliomethyl)-3-cephem-4-carboxylate;

7-{2-(2-Aminothiazole-4-yl)-2-[potassium-(2,2-dimethylacetate)oxyimino]acetamide}-3-(3-acetophenyl-1-imidazoliomethyl)-3-cephem-4-carboxylate;

7-{2-(2-Aminothiazole-4-yl)-2-[potassium-(2,2-dimethylacetate)oxyimino]acetamide}-3-(3-(4-hydroxyphenyl)-1-imidazoliomethyl)-3-cephem-4-carboxylate.

**2.** A process for preparing a cephalosporin compound of claim 1 or a pharmaceutically acceptable salt thereof which comprises reacting a compound of formula (II)

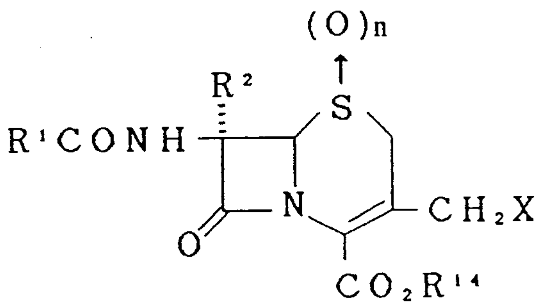

(II)

wherein $R^1$, $R^2$ and n are as defined in claim 1, $R^{14}$ is a hydrogen or metal atom, an ester residue, a salt-forming cation or an anionic charge when $COO^-$ forms an intra- or inter-molecular salt with a cation pair, X is a halogen atom or an acid residue which can be replaced by an imidazole compound (III), and a compound of formula (III)

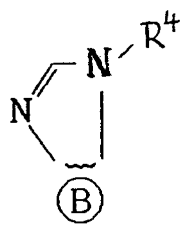

(III)

wherein $R^4$ is as defined in claim 1 and B is

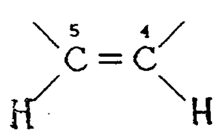

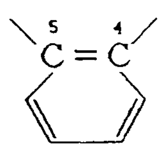

to obtain a cephalosporin compound having the following formula (IV) or a pharmaceutically acceptable salt thereof

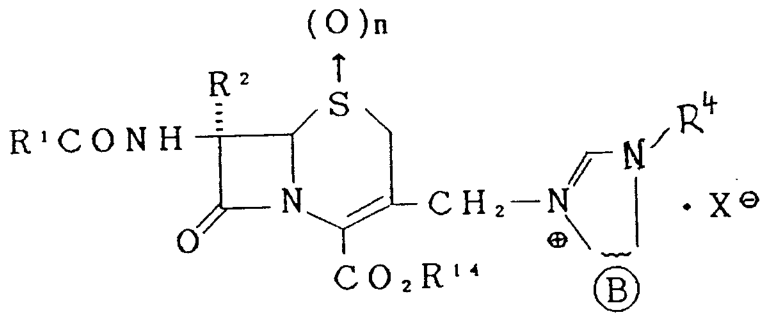

(IV)

wherein $R^1$, $R^2$, $R^4$, $R^{14}$, B, X and n are as defined above, and, if appropriate, further subjecting the compound of formula (IV) or a salt thereof to a reduction reaction or protective-group removing reaction.

**3.** A process for preparing a cephalosporin compound of claim 1 or a pharmaceutically acceptable salt thereof which comprises reacting a cephalosporin compound of formula (V)

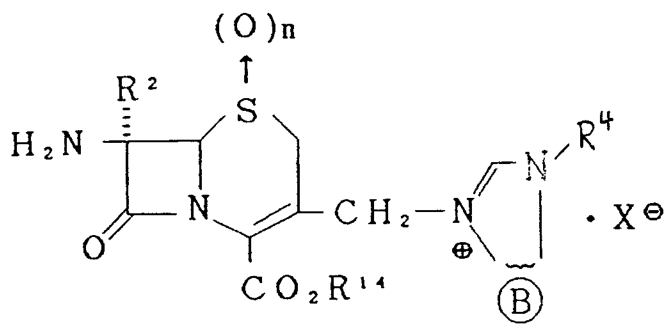

(V)

wherein $R^2$, $R^4$, $R^{14}$, B, X and n are as defined above, and a compound of formula (VI)

$R^1COOH$ (VI)

wherein R1 is as defined above, to obtain a cephalosporin compound having the following formula (IV) or a pharmaceutically acceptable salt thereof

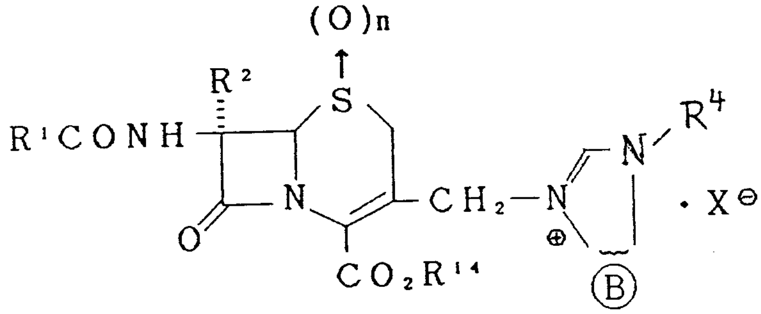

(IV)

wherein $R^1$, $R^2$, $R^4$, $R^{14}$, B, X and n are as defined above, and, if appropriate, further subjecting the compound of formula (IV) or a salt thereof to a reduction reaction or protective-group removing reaction.

**4.** A cephalosporin compound or a pharmaceutically acceptable salt thereof according to claim 1 for use as a pharmaceutical.

**5.** The use of a cephalosporin compound or a pharmaceutically acceptable salt thereof according to claim 1 in the manufacture of a medicament for preventing and treating bacterial infections.

6. A pharmaceutical composition comprising a cephalosporin compound or a pharmaceutically acceptable salt thereof according to claim 1 and a pharmaceutically acceptable carrier.

**Claims for the following Contracting State : AT**

1. A process for preparing a cephalosporin compound substituted by an imidazolium ring in 3-position of cephem having the formula (I) or (I') and the pharmaceutically acceptable salts thereof:

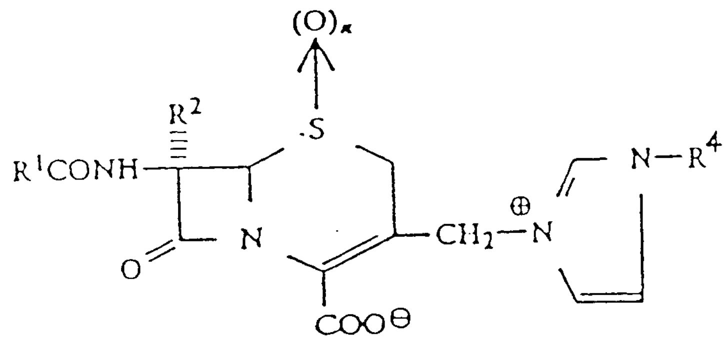

(I)

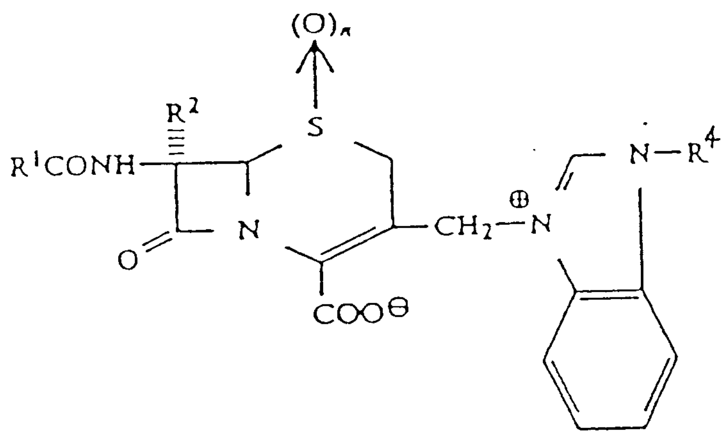

(I')

wherein

$R^1$ is

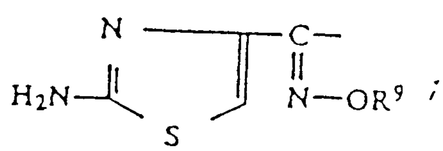

$R^2$ is hydrogen or methoxy;

$R^4$ is $C_2$-$C_{12}$ alkenyl, $C_2$-$C_{12}$ alkynyl, benzyl or phenyl;

$R^9$ is $C_1$-$C_6$ alkyl, $C_2$-$C_6$ alkenyl, $C_2$-$C_6$ alkynyl or a group of formula

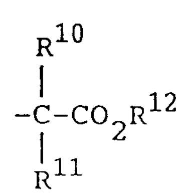

wherein $R^{10}$ and $R^{11}$ are the same or different and represent hydrogen, methyl or ethyl and

$R^{12}$ is hydrogen, an alkali metal, alkaline earth metal, organic amine base or protective group for carboxyl; and

n is 0 or 1;

said process comprising (a) reacting a compound of formula (II)

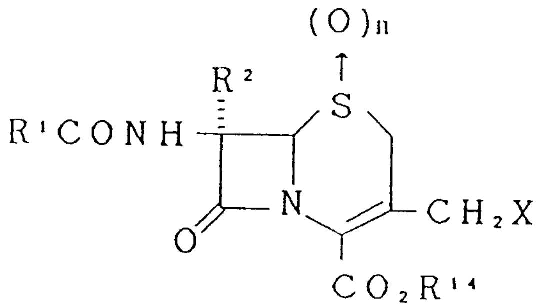

(II)

wherein $R^1$, $R^2$ and n are as defined above, $R^{14}$ is a hydrogen or metal atom, an ester residue, a salt-forming cation or an anionic charge when $COO^-$ forms an intra- or inter-molecular salt with a cation pair, X is a halogen atom or an acid residue which can be replaced by an imidazole compound (III), and a compound of formula (III)

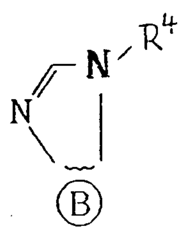

(III)

wherein $R^4$ is as defined above and B is

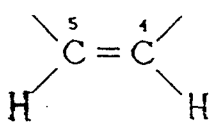

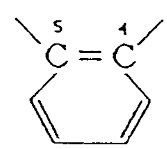

to obtain a cephalosporin compound having the following formula (IV) or a pharmaceutically acceptable salt thereof

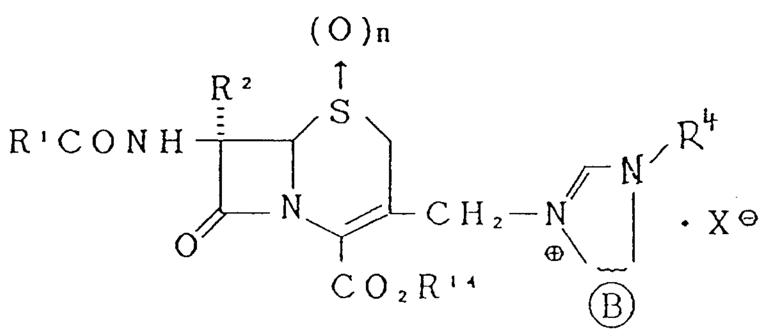

(IV)

wherein $R^1$, $R^2$, $R^4$, $R^{14}$, B, X and n are as defined above, and, if appropriate, further subjecting the compound of formula (IV) or a salt thereof to a reduction reaction or protective-group removing reaction.

or (b) reacting a cephalosporin compound of formula (V)

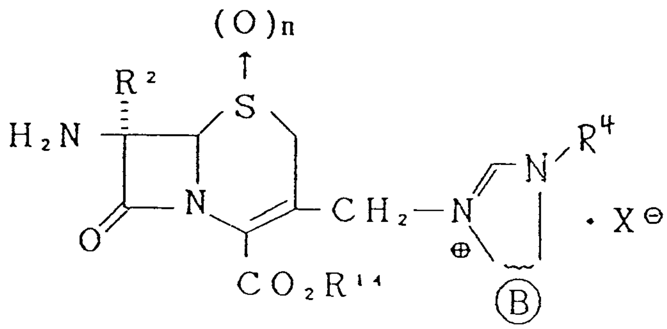

(V)

wherein $R^2$, $R^4$, $R^{14}$, B, X and n are as defined above, and a compound of formula (VI)

$R^1COOH$ (VI)

wherein $R^1$ is as defined above, to obtain a cephalosporin compound having the following formula (IV) or a pharmaceutically acceptable salt thereof

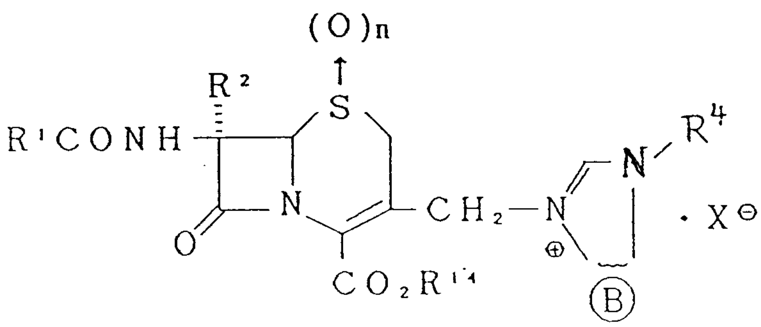

(IV)

wherein $R^1$, $R^2$, $R^4$, $R^{14}$, B, X and n are as defined above, and, if appropriate, further subjecting the compound of formula (IV) or a salt thereof to a reduction reaction or protective-group removing reaction.

**2.** Process for preparing 7-[2-(2-aminothiazole-4-yl)-2-(2-propenyloxyimino)-acetamide]-3-(3-acetophenyl-1-imidazoliomethyl)-3-cephem-4-carboxylate, comprising the reduction of the 1-oxido group of 7-[2-(2-propenyloxyimino)-2-(2-tritylaminothiazole-4-yl)acetamide]-3-(3-acetophenyl-1-imidazoliomethyl)-3-cephem-1-oxido-4-carboxylic acid p-methoxybenzyl ester iodide and the subsequent removal of the trityl group and the p-methoxybenzyl ester group.

**3.** Process for preparing 7-[2-(2-aminothiazole-4-yl)-2-(2-propenyloxyimino)-acetamide]-3-[3-(4-hydroxyphenyl-1-imidazoliomethyl]-3-cephem-4-carboxylate, comprising the reduction of the 1-oxido group of 7-[2-(2-propenyloxyimino)-2-(2-tritylaminothiazole-4-yl)acetamide]-3-[3-(4-hydroxyphenyl)-1-imidazoliomethyl]-3-cephem-1-oxido-4-carboxylic acid p-methoxybenzyl ester iodide and the subsequent removal of the trityl group and the p-methoxybenzyl ester group.

**4.** Process for preparing 7-[2-(2-aminothiazole-4-yl)-2-[potassium-(2,2-dimethylacetate)oxyimino]-acetamide}-3-(3-acetophenyl-1-imidazoliomethyl)-3-cephem-4-carboxylate, comprising the reduction of the 1-oxido group of 7-[2-(2-tert-butoxycarbonyl)prop-2-oxyimino-2-(2-tritylaminothiazole-4-yl)-acetamide]-3-(3-acetophenyl-1-imidazoliomethyl)-3-cephem-1-oxido-4-carboxylic acid p-methoxybenzyl ester iodide, the subsequent removal of the trityl group, the saponification of the ester groups with acid and the salification of the resulting product with a potassium base.

5. Process for preparing 7-{2-(2-aminothiazole-4-yl)-2-[potassium-(2,2-dimethylacetate)oxyimino]-acetamide}-3-[3-(4-hydroxyphenyl)-1-imidazoliomethyl]-3-cephem-4-carboxylate, comprising the reduction of the 1-oxido group of 7-[2-(2-tert-butoxycarbonyl)prop-2-oxyimino-2-(2-tritylaminothiazole-4-yl)-acetamide]-3-[3-(4-hydroxyphenyl)-1-imidazoliomethyl]-3-cephem-1-oxido-4-carboxylic acid p-methoxybenzyl ester iodide, the subsequent removal of the trityl group, the saponification of the ester groups with acid and the salification of the resulting product with a potassium base.

6. The use of a cephalosporin compound or a pharmaceutically acceptable salt thereof which is obtainable according to the process of any one of claims 1 to 5 in the manufacture of a medicament for preventing and treating bacterial infections.

**Patentansprüche**
**Patentansprüche für folgende Vertragsstaaten : BE, CH, DE, FR, GB, IT, LI, NL, SE**

1. Cephalosporinverbindung, die an der 3-Stellung von Cephem durch einen Imidazoliumring substituiert ist, mit der Formel (I) oder (I') und, die pharmazeutisch annehmbaren Salze davon:

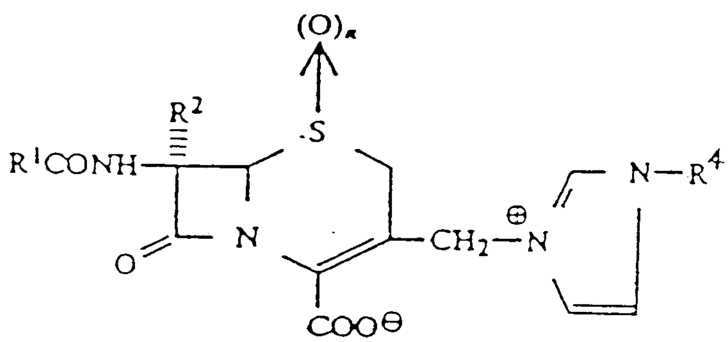
(I)

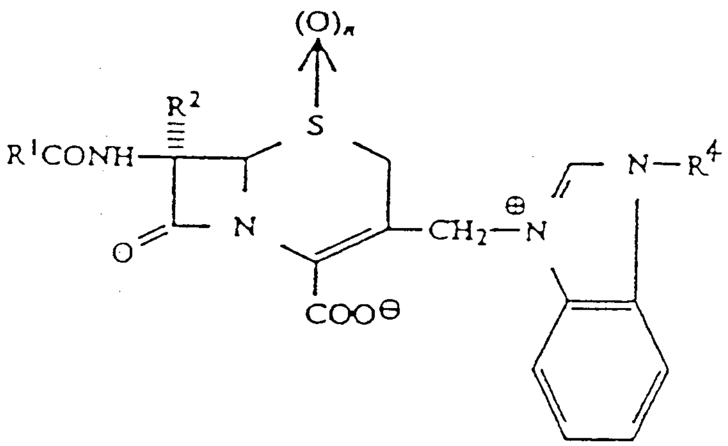
(I')

worin
$R^1$

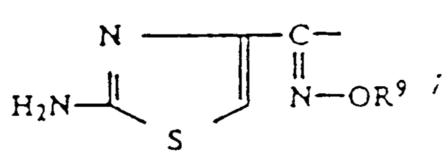

ist;
$R^2$ Wasserstoff oder Methoxy ist;

$R^4$ $C_2$-$C_{12}$-Alkenyl, $C_2$-$C_{12}$-Alkinyl, Benzyl oder Phenyl ist;
$R^9$ $C_1$-$C_6$-Alkyl, $C_2$-$C_6$-Alkenyl, $C_2$-$C_6$-Alkinyl oder eine Gruppe der Formel

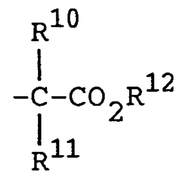

ist, worin $R^{10}$ und $R^{11}$ gleich oder verschieden sind und Wasserstoff, Methyl oder Ethyl darstellen und $R^{12}$ Wasserstoff, ein Alkalimetall, ein Erdalkalimetall, eine organische Aminbase oder eine Schutzgruppe für Carboxyl ist;

und n 0 oder 1 ist; die folgenden Verbindungen werden ebenfalls beansprucht

7-[2-(2-Aminothiazol-4-yl)-2-(2-propenyloxyimino)-acetamid]-3-(3-acetophenyl-1-imidazoliomethyl)-3-cephem-4-carboxylat;

7-[2-(2-Aminothiazol-4-yl)-2-(2-propenyloxyimino)-acetamid]-3-(3-(4-hydroxyphenyl)-1-imidazoliomethyl)-3-cephem-4-carboxylat;

7-{2-(2-Aminothiazol-4-yl)-2-[Kalium-(2,2-dimethylacetat)oxyimino]acetamid}-3-(3-acetophenyl-1-imidazoliomethyl)-3-cephem-4-carboxylat;

7-{2-(2-Aminothiazol-4-yl)-2-[Kalium-(2,2-dimethylacetat)oxyimino]acetamid}-3-(3-(4-hydroxyphenyl)-1-imidazoliomethyl)-3-cephem-4-carboxylat.

**2.** Verfahren zur Herstellung einer Cephalosporinverbindung nach Anspruch 1 oder eines pharmazeutisch annehmbaren Salzes davon, umfassend das Umsetzen einer Verbindung der Formel (II)

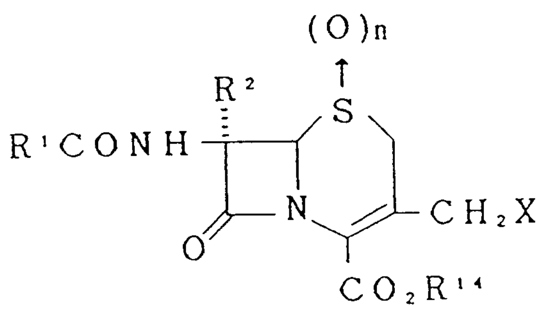

(II)

worin $R^1$, $R^2$ und n wie in Anspruch 1 definiert sind, $R^{14}$ Wasserstoff oder ein Metallatom, ein Ester-Rest, ein salzbildendes Kation oder, wenn $COO^-$ ein intra- oder intermolekulares Salz mit einem Kationenpaar bildet, eine anionische Ladung ist, X ein Halogenatom oder ein Säurerest, welches bzw. welcher durch eine Imidazolverbindung (III) ersetzt werden kann, ist, und einer Verbindung der Formel (III)

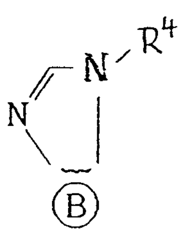

(III)

worin $R^4$ wie in Anspruch 1 definiert ist und B

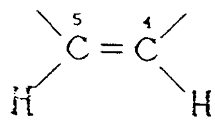

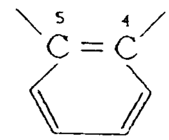

ist, um eine Cephalosporinverbindung, die die folgende Formel (IV) aufweist, oder ein pharmazeutisch annehmbares Salz davon zu erhalten

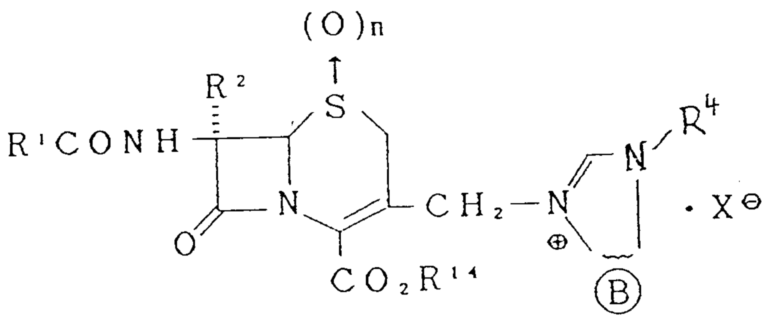
(IV)

worin $R^1$, $R^2$, $R^4$, $R^{14}$, B, X und n wie oben definiert sind, und, falls angezeigt, das weitere Unterziehen der Verbindung der Formel (IV) oder eines Salzes davon unter eine Reduktionsreaktion oder eine Schutzgruppen-entfernende Reaktion.

**3.** Verfahren zur Herstellung einer Cephalosporinverbindung nach Anspruch 1 oder eines pharmazeutisch annehmbaren Salzes davon, umfassend das Umsetzen einer Cephalosporinverbindung der Formel (V)

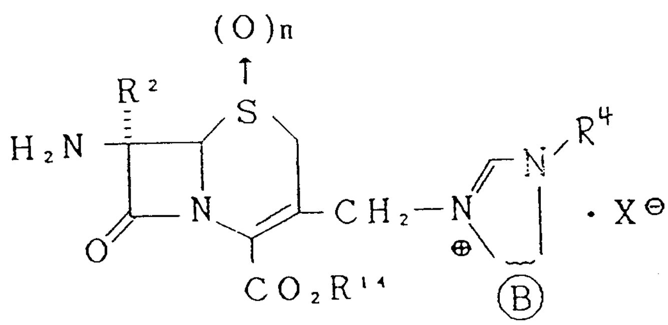
(V)

worin $R^2$, $R^4$, $R^{14}$, B, X und n wie oben definiert sind, und einer Verbindung der Formel (VI)

$R^1COOH$ (VI)

worin $R^1$ wie oben definiert ist, um eine Cephalosporinverbindung mit der folgenden Formel (IV) oder ein pharmazeutisch annehmbares Salz davon zu erhalten

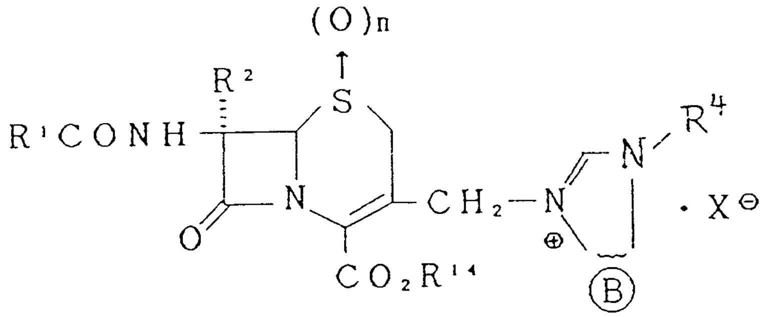

(IV)

worin $R^1$, $R^2$, $R^4$, $R^{14}$, B, X und n wie oben definiert sind, und, falls angezeigt, das weitere Unterziehen der Verbindung der Formel (IV) oder eines Salzes davon unter eine Reduktionsreaktion oder Schutzgruppen-entfernende Reaktion.

**4.** Cephalosporinverbindung oder ein pharmazeutisch annehmbares Salz davon gemäß Anspruch 1 zur Verwendung als Arzneistoff.

**5.** Verwendung einer Cephalosporinverbindung oder eines pharmazeutisch annehmbaren Salzes davon nach Anspruch 1 bei der Herstellung eines Medikaments zur Verhütung und Behandlung von bakteriellen Infektionen.

**6.** Pharmazeutische Zusammensetzung, umfassend eine Cephalosporinverbindung oder ein pharmazeutisch annehmbares Salz davon nach Anspruch 1 und einen pharmazeutisch annehmbaren Träger.

**Patentansprüche für folgenden Vertragsstaat : AT**

**1.** Verfahren zur Herstellung einer Cephalosporinverbindung, die an der 3-Stellung von Cephem durch einen Imidazoliumring substituiert ist, mit der Formel (I) oder (I'), und der pharmazeutisch annehmbaren Salze davon:

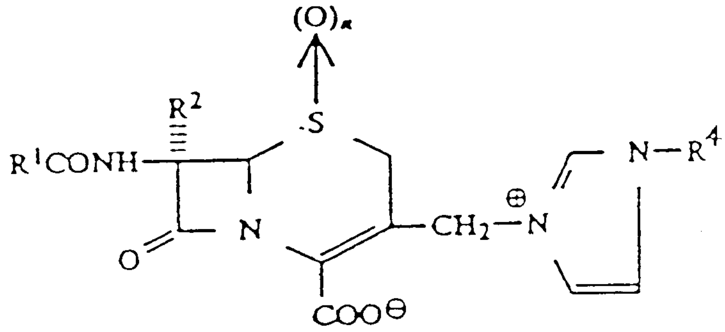

(I)

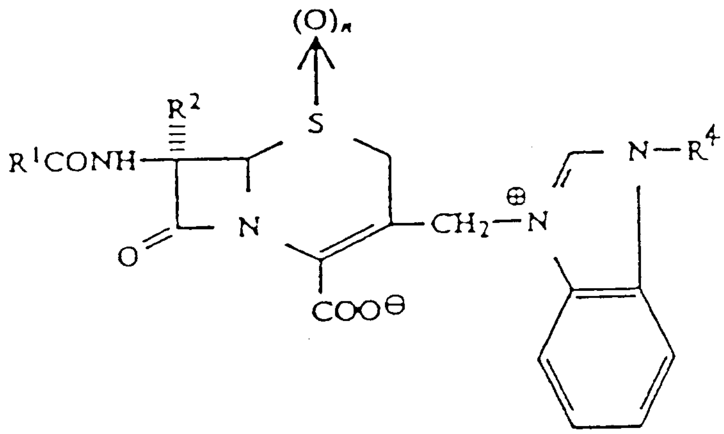

(I')

worin

$R^1$

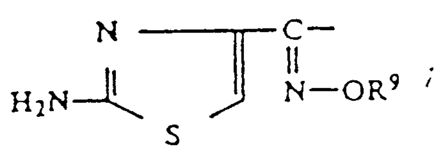

ist;

- $R^2$ Wasserstoff oder Methoxy ist;
- $R^4$ $C_2$-$C_{12}$-Alkenyl, $C_2$-$C_{12}$-Alkinyl, Benzyl oder Phenyl ist;
- $R^9$ $C_1$-$C_6$-Alkyl, $C_2$-$C_6$-Alkenyl, $C_2$-$C_6$-Alkinyl oder eine Gruppe der Formel

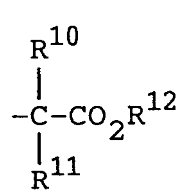

ist, worin $R^{10}$ und $R^{11}$ gleich oder verschieden sind und Wasserstoff, Methyl oder Ethyl darstellen und $R^{12}$ Wasserstoff, ein Alkalimetall, ein Erdalkalimetall, eine organische Aminbase oder eine Schutzgruppe für Carboxyl ist;

und n 0 oder 1 ist;

wobei dieses Verfahren umfaßt

(a) das Umsetzen einer Verbindung der Formel (II)

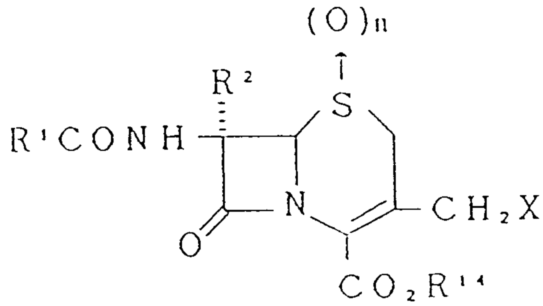

(II)

worin $R^1$, $R^2$ und n wie oben definiert sind, $R^{14}$ Wasserstoff oder ein Metallatom, ein Ester-Rest, ein salzbildendes Kation oder, wenn $COO^-$ ein intra- oder intermolekulares Salz mit einem Kationenpaar bildet, eine anionische Ladung ist, X ein Halogenatom oder ein Säurerest, welches bzw. welcher durch eine Imidazolverbindung (III) ersetzt werden kann, ist, und einer Verbindung der Formel (III)

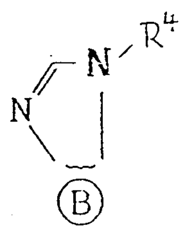

(III)

worin $R^4$ wie oben definiert ist und B

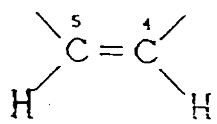

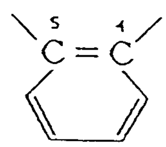

ist, um eine Cephalosporinverbindung, die die folgende Formel (IV) aufweist, oder ein pharmazeutisch annehmbares Salz davon zu erhalten

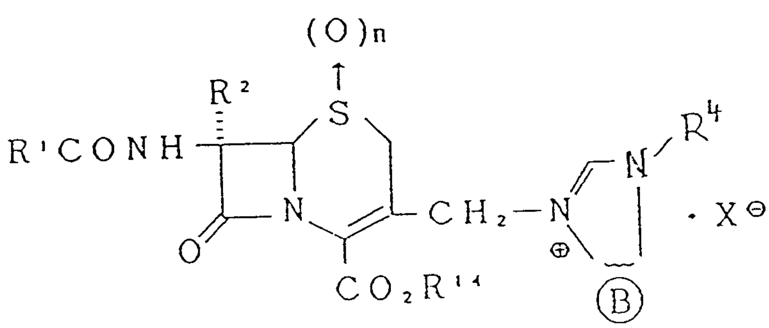

(IV)

worin $R^1$, $R^2$, $R^4$, $R^{14}$, B, X und n wie oben definiert sind, und, falls angezeigt, das weitere Unterziehen der Verbindung der Formel (IV) oder eines Salzes davon unter eine Reduktionsreaktion oder eine Schutzgruppen-entfernende Reaktion;

oder (b) das Umsetzen einer Cephalosporinverbindung der Formel (V)

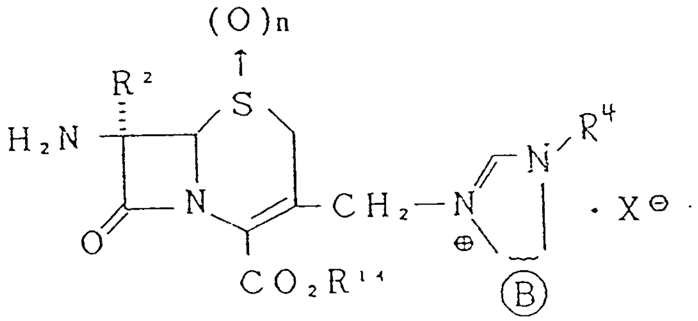

(V)

worin $R^2$, $R^4$, $R^{14}$, B, X und n wie oben definiert sind, und einer Verbindung der Formel (VI)

$R^1COOH$ (VI)

worin $R^1$ wie oben definiert ist, um eine Cephalosporin-Verbindung mit der folgenden Formel (IV) oder ein pharmazeutisch annehmbares Salz davon zu erhalten

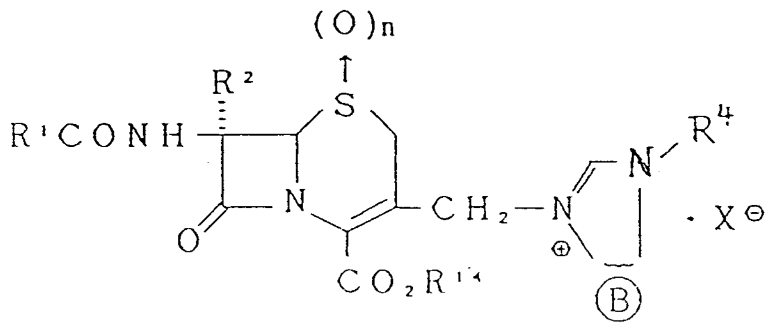

(IV)

worin $R^1$, $R^2$, $R^4$, $R^{14}$, B, X und n wie oben definiert sind, und, falls angezeigt, das weitere Unterziehen der Verbindung der Formel (IV) oder eines Salzes davon unter eine Reduktionsreaktion oder Schutzgruppen-entfernende Reaktion.

2. Verfahren zur Herstellung von 7-[2-(2-Aminothiazol-4-yl)-2-(2-propenyloxyimino)-acetamid]-3-(3-acetophenyl-1-iminozoliomethyl)-3-cephem-4-carboxylat, umfassend die Reduktion der 1-Oxidogruppe von 7-[2-(2-Propenyloxyimino)-2-(2-tritylaminothiazol-4-yl)acetamid]-3-(3-acetophenyl-1-imidazoliomethyl)-3-cephem-1-oxido-4-carbonsäure-p-methoxybenzylesteriodid und die anschließende Entfernung der Tritylgruppe und der p-Methoxybenzylestergruppe.

3. Verfahren zur Herstellung von 7-[2(2-Aminothiazol-4-yl)-2-(2-propenyloxyimino)-acetamid]-3-[3-(4-hydroxyphenyl-1-imidazoliomethyl]-3-cephem-4-carboxylat,umfassend die Reduktion der 1-Oxidogruppe von 7-[2-(2-Propenyloxyimino)-2-(2-tritylaminothiazol-4-yl)acetamid]-3-[3-(4-hydroxyphenyl)-1-imidazoliomethyl]-3-cephem-1-oxido-4-carbonsäure-p-methoxybenzylesteriodid und die darauffolgende Entfernung der Tritylgruppe und der p-Methoxybenzylestergruppe.

4. Verfahren zur Herstellung von 7{2-(2-Aminothiazol-4-yl)-2-[Kalium-(2,2-dimethylacetat)oxyimino]-acetamid}-3-(3-acetophenyl-1-imidazoliomethyl)-3-cephem-4-carboxylat, umfassend die Reduktion der 1-Oxidogruppe von 7-[2-(2-tert-Butoxycarbonyl)prop-2-oxyimino-2-(2-tritylaminothiazol-4-yl)acetamid]-3-(3-acetophenyl-1-imidazoliomethyl)-3-cephem-1-oxido-4-carbonsäure-p-methoxybenzylesteriodid, die anschließende Entfernung der Tritylgruppe, die Verseifung der Estergruppen mit Säure und die Bildung des Salzes des resultierenden Produkts mit einer Kaliumbase.

5. Verfahren zur Herstellung von 7-{2-(2-Aminothiazol-4-yl)-2-[Kalium-(2,2-dimethylacetat)oxyimino]-acetamid}-3-[3-(4-hydroxyphenyl)-1-imidazoliomethyl]-3-cephem-4-carboxylat, umfassend die Reduktion der 1-Oxidogruppe von 7-[2-(2-tert-Butoxycarbonyl)prop-2-oxyimino-2-(2-trityl-aminothiazol-4-yl)-acetamide]-3-[3-(4-hydroxyphenyl)-1-imidazoliomethyl]-3-cephem-1-oxido-4-carbonsäure-p-methoxybenzylesteriodid, die anschließende Entfernung der Tritylgruppe, die Verseifung der Estergruppen mit Säure und die Bildung des Salzes des resultierenden Produkts mit einer Kaliumbase.

6. Verwendung einer Cephalosporinverbindung oder eines pharmazeutisch annehmbaren Salzes davon, das nach dem Verfahren von irgendeinem der Ansprüche 1 bis 5 erhältlich ist, bei der Herstellung eines Medikaments zur Verhütung und Behandlung von bakteriellen Infektionen.

**Revendications**

**Revendications pour les Etats contractants suivants : BE, CH, DE, FR, GB, IT, LI, NL, SE**

1. Céphalosporine substituée par un cycle d' imidazolium à la position 3 du céphème, répondant la formule (I) ou (I'), et ses sels pharmaceutiquement acceptables :

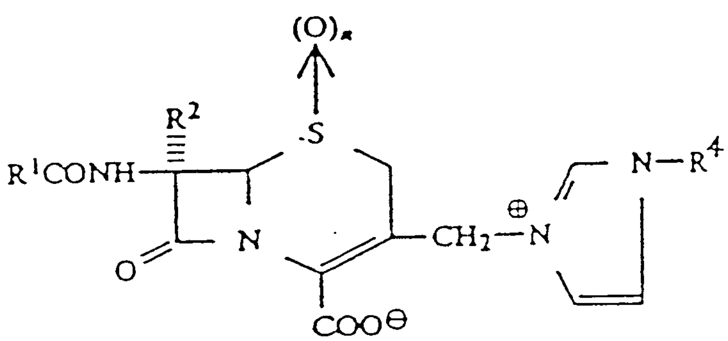

(I)

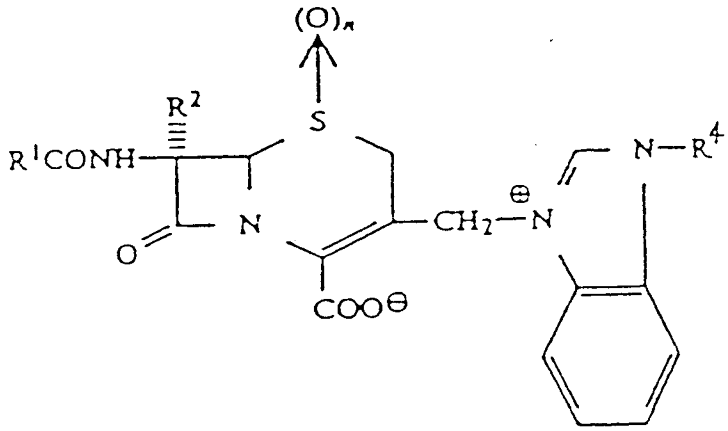

(I')

où

R[1] est

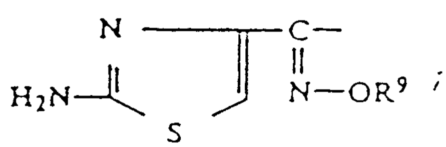

$R^2$ est l'hydrogène ou le groupe méthoxy ;

$R^4$ est un groupe alcényle en $C_2$-$C_{12}$, alcynyle en $C_2$-$C_{12}$, benzyle ou phényle ;

$R^9$ est un groupe alkyle en $C_1$-$C_6$, alcényle en $C_2$-$C_6$, alcynyle en $C_2$-$C_6$ ou un groupe de formule

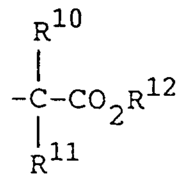

où $R^{10}$ et $R^{11}$ sont identiques ou différents et représentent l'hydrogène, un groupe méthyle ou éthyle, et $R^{12}$ est l'hydrogène, un métal alcalin, un métal alcalino-terreux, une base du type amine organique ou un groupe protecteur du groupe carboxyle ; et

n est 0 ou 1 ;

les composés suivants sont également revendiqués

- 7-[2-(2-aminothiazole-4-yl)-2-(2-propényloxyimino)acétamido]-3-(3-acétophényl-1-imidazoliométhyl)-3-céphème-4-carboxylate ;
- 7-[2-(2-aminothiazole-4-yl)-2-(2-propényloxyimino)acétamido]-3-(3-(4-hydroxyphényl)-1-imidazoliométhyl)-3-céphème-4-carboxylate ;
- 7-{2-(2-aminothiazole-4-yl)-2-[(2,2-diméthylacétate de potassium)oxyimino]acétamido}-3-(3-acétophényl-1-imidazoliométhyl)-3-céphème-4-carboxylate ;
- 7-{2-(2-aminothiazole-4-yl)-2-[(2,2-diméthylacétate de potassium)oxyimino]acétamido}-3-(3-(4-hydroxyphényl)-1-imidazoliométhyl)-3-céphème-4-carboxylate.

**2.** Procédé de préparation d'une céphalosporine de la revendication 1 ou d'un sel pharmaceutiquement acceptable de celle-ci, qui consiste à faire réagir un composé de formule (II)

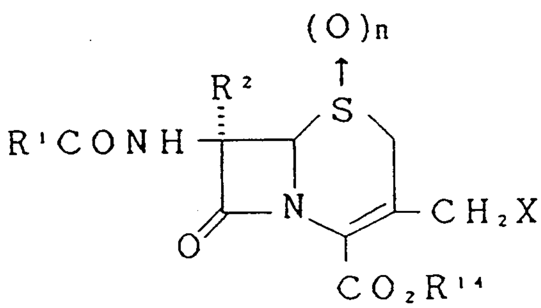

(II)

où $R^1$, $R^2$ et n sont tels que définis dans la revendication 1, $R^{14}$ est un atome d'hydrogène ou de métal, un résidu d'ester, un cation de salification ou une charge anionique lorsque $COO^-$ forme un sel intra- ou intermoléculaire avec un doublet cationique, X est un atome d'halogène ou un résidu d'acide qui peut être remplacé par un composé d'imidazole (III), et un composé de formule (III)

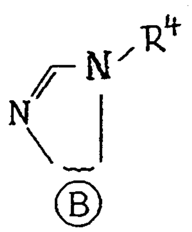

(III)

où $R^4$ est tel que défini dans la revendication 1 et B est

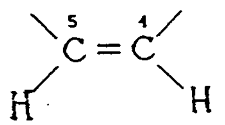

ou

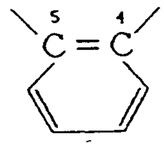

pour obtenir une céphalosporine répondant à la formule (IV) suivante ou un sel pharmaceutiquement acceptable de celle-ci

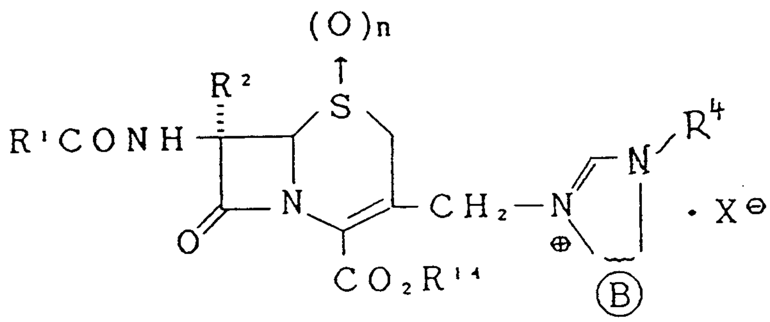

(IV)

où $R^1$, $R^2$, $R^4$, $R^{14}$, B, X et n sont tels que définis ci-dessus et, facultativement, à soumettre de plus le composé de formule (IV) ou son sel à une réaction de réduction ou une réaction d'élimination de groupe protecteur.

**3.** Procédé de préparation d'une céphalosporine de la revendication 1 ou d'un sel pharmaceutiquement acceptable de celle-ci, qui consiste à faire réagir une céphalosporine de formule (V)

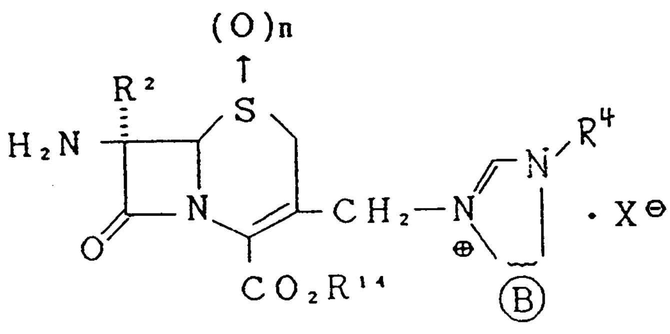

(V)

où $R^2$, $R^4$, $R^{14}$, B, X et n sont tels que définis ci-dessus, et un composé de formule (VI)

$R^1COOH$ (VI)

où $R^1$ est tel que défini ci-dessus, pour obtenir une céphalosporine répondant à la formule (IV) suivante ou un sel pharmaceutiquement acceptable de celle-ci

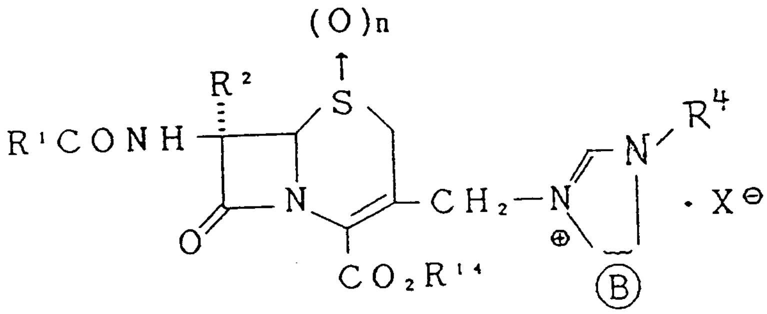

(IV)

où $R^1$, $R^2$, $R^4$, $R^{14}$, B, X et n sont tels que définis ci-dessus, et, facultativement, à soumettre de plus le composé de formule (IV) ou son sel à une réaction de réduction ou une réaction d'élimination de groupe protecteur.

**4.** Céphalosporine ou sel pharmaceutiquement acceptable de celle-ci selon la revendication 1, pour son utilisation comme agent pharmaceutique.

**5.** Utilisation d'une céphalosporine ou d'un sel pharmaceutiquement acceptable de celle-ci selon la revendication 1 dans la fabrication d'un médicament destiné à prévenir ou traiter des infections bactériennes.

**6.** Composition pharmaceutique comprenant une céphalosporine ou un sel pharmaceutiquement acceptable de celle-ci selon la revendication 1 et un support pharmaceutiquement acceptable.

**Revendications pour l'Etat contractant suivant : AT**

**1.** Procédé de préparation d'une céphalosporine substituée par un cycle d'imidazolium à la position 3 du céphème, répondant la formule (I) ou (I'), et ses sels pharmaceutiquement acceptables :

(I)

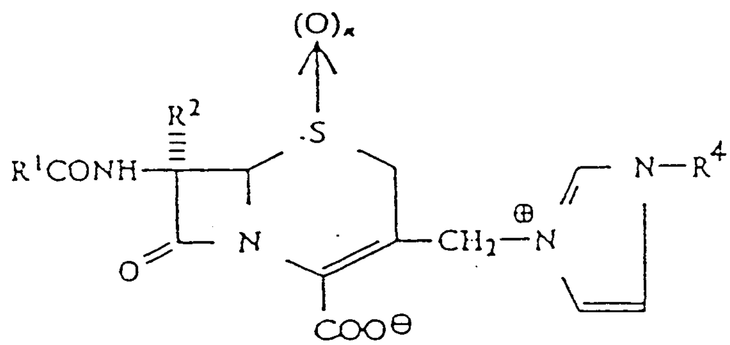

(I')

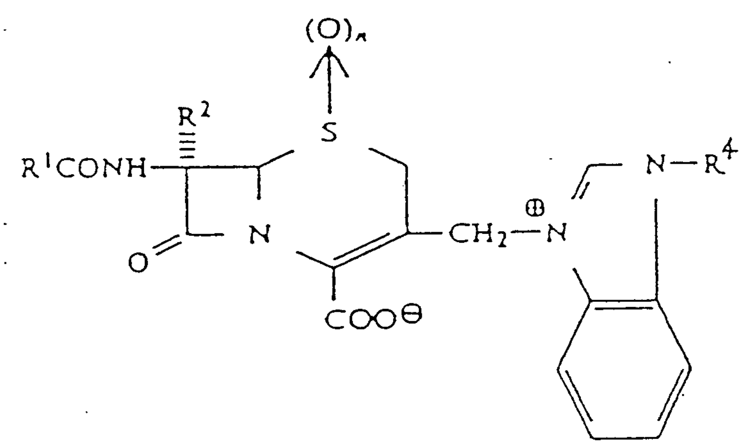

où

$R^1$ est

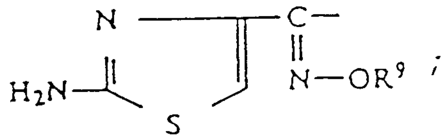

$R^2$ est l'hydrogène ou le groupe méthoxy ;

$R^4$ est un groupe alcényle en $C_2$-$C_{12}$, alcynyle en $C_2$-$C_{12}$, benzyle ou phényle ;

$R^9$ est un groupe alkyle en $C_1$-$C_6$, alcényle en $C_2$-$C_6$,alcynyle en $C_2$-$C_6$ ou un groupe de formule

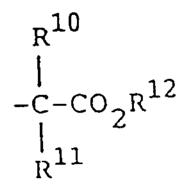

où $R^{10}$ et $R^{11}$ sont identiques ou différents et représentent l'hydrogène, un groupe méthyle ou éthyle, et $R^{12}$ est l'hydrogène, un métal alcalin, un métal alcalino-terreux, une base du type amine organique ou un groupe protecteur du groupe carboxyle ; et

n est 0 ou 1.

ledit procédé consistant (a) à faire réagir un composé de formule (II)

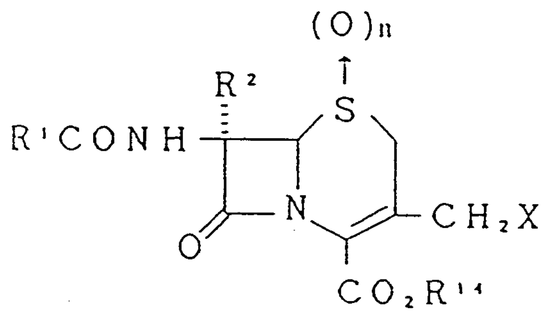

(II)

où $R^1$, $R^2$ et n sont tels que définis ci-dessus, $R^{14}$ est un atome d'hydrogène ou de métal, un résidu d'ester, un cation de salification ou une charge anionique lorsque $COO^-$ forme un sel intra- ou intermoléculaire avec un doublet cationique, X est un atome d'halogène ou un résidu d'acide qui peut être remplacé par un composé d'imidazole (III), et un composé de formule (III)

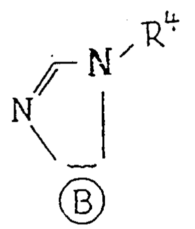

(III)

où $R^4$ est tel que défini ci-dessus et B est

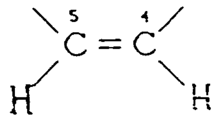

ou

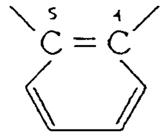

pour obtenir une céphalosporine répondant à la formule (IV) suivante ou un sel pharmaceutiquement acceptable de celle-ci

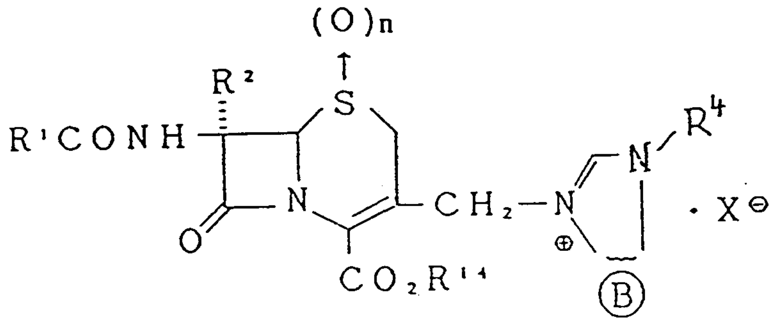

(IV)

où $R^1$, $R^2$, $R^4$, $R^{14}$, B, X et n sont tels que définis ci-dessus et, facultativement, à soumettre de plus le composé de formule (IV) ou son sel à une réaction de réduction ou une réaction d'élimination de groupe protecteur,
ou (b) à faire réagir une céphalosporine de formule (V)

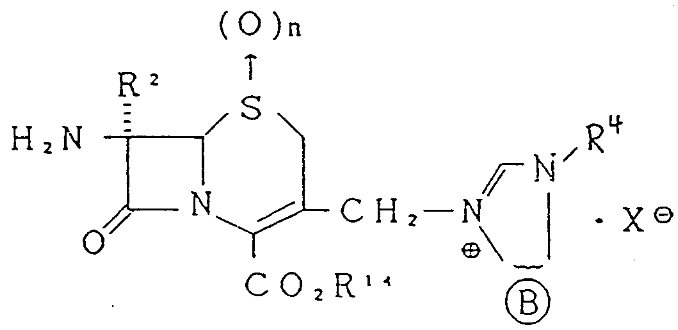

(V)

où $R^2$, $R^4$, $R^{14}$, B, X et n sont tels que définis ci-dessus, et un composé de formule (VI)

$R^1COOH$ (VI)

où $R^1$ est tel que défini ci-dessus, pour obtenir une céphalosporine répondant à la formule (IV) suivante ou un sel pharmaceutiquement acceptable de celle-ci

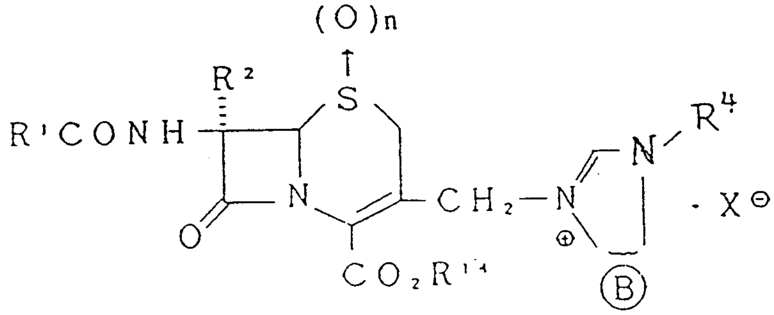

(IV)

où $R^1$, $R^2$, $R^4$, $R^{14}$, B, X et n sont tels que définis ci-dessus, et, facultativement, à soumettre de plus le composé de formule (IV) ou son sel à une réaction de réduction ou une réaction d'élimination de groupe protecteur.

**2.** Procédé de préparation du 7-[2-(2-aminothiazole-4-yl)-2-(2-propényloxyimino)acétamido]-3-(3-acétophényl-1-imidazoliométhyl)-3-céphème-4-carboxylate, comprenant la réduction du groupe 1-oxydo de l'iodure d'ester *p*-méthoxybenzylique d'acide 7-[2-(2-propényloxyimino)-2-(2-tritylaminothiazole-4-yl)-acétamido]-3-(3-acétophényl-1-imidazoliométhyl)-3-céphème-1-oxydo-4-carboxylique et l'élimination subséquente du groupe trityle et du groupe ester *p*-méthoxybenzylique.

**3.** Procédé de préparation du 7-[2-(2-aminothiazole-4-yl)-2-(2-propényloxyimino)acétamido]-3-[3-(4-hydroxyphényl-1-imidazoliométhyl]-3-céphème-4-carboxylate,comprenant la réduction du groupe 1-oxydo de l'iodure d'ester *p*-méthoxybenzylique d'acide 7-[2-(2-propényloxyimino)-2-(2-tritylaminothiazole-4-yl)acétamido]-3-(3-(4-hydroxyphényl)-1-imidazoliométhyl]-3-céphème-1-oxydo-4-carboxylique et l'élimination subséquente du groupe trityle et du groupe ester *p*-méthoxybenzylique.

**4.** Procédé de préparation du 7-{2-(2-aminothiazole-4-yl)-2-[(2,2-diméthylacétate de potassium)oxyimino]-acétamido}-3-(3-acétophényl-1-imidazoliométhyl)-3-céphème-4-carboxylate, comprenant la réduction du groupe 1-oxydo de l'iodure d'ester *p*-méthoxybenzylique d'acide 7-[2-(2-*tert*-butoxycarbonyl)prop-2-oxyimino-2-(2-tritylaminothiazole-4-yl)acétamido]-3-(3-acétophényl-1-imidazoliométhyl)-3-céphème-1-oxydo-4-carboxylique, l'élimination subséquente du groupe trityle, la saponification des groupes ester avec un acide et la salification du produit résultant avec une base potassique.

**5.** Procédé de préparation du 7-{2-(2-aminothiazole-4-yl)-2-[(2,2-diméthylacétate de potassium)oxyimino]-acétamido}-3-[3-(4-hydroxyphényl)-1-imidazoliométhyl)-3-céphème-4-carboxylate, comprenant la réduction du groupe 1-oxydo de l'iodure d'ester *p*-méthoxybenzylique d'acide 7-[2-(2-*tert*-butoxycarbonyl)-prop-2-oxyimino-2-(2-tritylaminothiazole-4-yl)acétamido]-3-[3-(4-hydroxyphényl)-1-imidazoliométhyl)-3-céphème-1-oxydo-4-carboxylique, l'élimination subséquente du groupe trityle, la saponification des groupes ester avec un acide et la salification du produit résultant avec une base potassique.

**6.** Utilisation d'une céphalosporine ou d'un sel pharmaceutiquement acceptable de celle-ci, qui peut être obtenu selon le procédé de l'une quelconque des revendications 1 à 5, dans la fabrication d'un médicament destiné à prévenir ou traiter des infections bactériennes.